# EUROPEAN PATENT APPLICATION

(11) **EP 1 731 131 A1**
(43) Date of publication of application: **13.12.2006**
(21) Application number: 05727806.1
(22) Date of filing: 28.03.2005
(51) Int. Cl.: A61K 6/00

(54) **HGF PRODUCTION ACCELERATOR CONTAINING HEPARIN-LIKE OLIGOSACCHARIDE**

(30) Priority: 29.03.2004 JP 2004097047
(71) Applicant: Kringle Pharma Inc., Toyonaka-shi, Osaka 560-0082 (JP); Nakamura, Toshikazu, Kyoto 606-8333 (JP)
(72) Inventor: NAKAMURA, Toshikazu, Kyoto-shi, Kyoto 6068333 (JP); MATSUMOTO, Kunio, Mino-shi, Osaka 5620031 (JP); FUKUTA, Kazuhiro, Mino-shi, Osaka 5620031 (JP)
(74) Representative: Best, Michael
(86) International application number: PCT/JP2005/005741
(87) International publication number: WO 2005/092348

(57) **Abstract**

The present invention aims to provide an agent for promoting HGF production comprising, as an effective ingredient, a disaccharide comprised of an uronic acid residue (wherein an uronic acid means an iduronic acid or a glucuronic acid, and has the same meaning hereinafter) and a glucosamine residue that are connected by α1,4-glycosidic linkage or β1,4-glycosidic linkage, or an oligosaccharide of tri- to hexadeca-saccharides having a structure in which uronic acid residues and glucosamine residues are alternately and repeatedly connected by α1,4-glycosidic linkage or β1,4-glycosidic linkage, wherein at least one hydroxy group of the uronic acid residues and/or the glucosamine residues may be sulfated, alkylated, acylated or aminated, and/or the amino group at position 2 of at least one of the glucosamine residue(s) may be sulfated, alkylated or acylated, or a salt thereof. The agent of the present invention for promoting HGF production is useful to promote healing of damaged tissues or organs of a living body.

## Description

### TECHNICAL FIELD

The present invention relates to an agent for promoting HGF production comprising a sugar chain compound such as low-molecular-weight oligosaccharides having a heparin structure, or a salt thereof.

### BACKGROUND ART

Much attention has been given to HGF (hepatocyte growth factor) as a regeneration factor or a therapeutic factor.

HGF is a protein which was found on the basis of mitogenic activity on hepatocytes, however, subsequent studies have clarified that HGF exerts mitogenic activity on many epithelial cells in addition to hepatocytes and on some kinds of mesenchymal cells.

It is also known that HGF exerts not only mitogenic activity but also various activities such as motogenic, morphogenic, anti-apoptotic, and neovascularization activities (Matsumoto, K et al., Kidney Int., 2001, vol.59, p.2023-2038). On the basis of these pharmacological actions of HGF, its development as the following agents has been expected: cirrhosis treating agents, renal disease treating agents, epithelial cell growth promoting agents, anti-cancer agents, side effect preventing agents in cancer therapy, lung disease treating agents, stomach or duodenum damage treating agents, cerebral nerve disease treating agents, side effect preventing agents of immune suppression, collagen degradation promoting agents, cartilage disease treating agents, arterial disease treating agents, pulmonary fibrosis treating agents, hepatic disease treating agents, blood coagulation abnormality treating agents, plasma low protein treating agents, wound healing agents, neuropathy improving agents, hematopoietic stem cell increasing agents, or hair growth promoting agents (see JP-A-18028/1992, JP-A-49246/1992, EP-A-492614, JP-A-25010/1994, WO 03/8821, JP-A-172207/1994, JP-A-89869/1995, JP-A-40934/1994, WO 94/2165, JP-A -40935/1994, JP-A-56692/1994, JP-A-41429/1995, WO 93/3061, and JP-A-213721/1993).

HGF is produced mainly by mesenchymal cells, and its production amount is increased in response to organ damages. For example, when injury occurs in the liver, HGF production is increased in liver vascular endothelial cells and non-hepatocytes such as Kupffer cells and Ito cells. On the other hand, at this time, expression of HGF is increased also in other organs such as non-injured lung, spleen, and kidney, and thus HGF level in the blood is increased. This suggests that factors inducing expression of HGF in response to organ injuries are present in the blood, and such factors are termed collectively as "injurin" (Matsumoto, K et al., Proc. Natl. Acad. Sci. USA., 1992, vol.89, p.3800 to 3804).

Injurin is not a single substance, and for example, cytokines such as interleukin-1, FGF (fibroblast growth factor) and PDGF (platelet-derived growth factor), and autacoids such as prostaglandins are identified as injurin. These factors promote HGF production by increasing expression of HGF mRNA. On the other hand, during investigation of injurin, it was found that heparin and heparan sulfate also have an activity to promote HGF production (Matsumoto, K. et al,.J.Biochem., 1993, vol.114, p.820 to 826).

It is suggested that heparin and heparan sulfate do not increase expression of HGF mRNA, but act on step(s) after transcription process. However, detailed mechanism is unknown.

Heparin and heparan sulfate are a member of polysaccharide group known as glycosaminoglycans (GAG). Heparin and heparan sulfate are polysaccharides constructed by the repetition of disaccharide units composed of D-glucosamine and uronic acid (Sugahara, K et al., IUBMB Life, 2002, vol. 54, p.163 to 175).

A molecular weight of heparin and heparan sulfate is not uniform, and substances having a molecular weight of around 5000 to 30000 are present therein in a mixed form. The carbon atom at position 6 of the D-glucosamine residues partially undergoes O-sulfation, and the amino group of D-glucosamine residues may undergo N-acetylation or N-sulfation. The uronic acid residues exist as either L-iduronic acid or D-glucronic acid. Most of the uronic acid residues exist as L-iduronic acid in heparin, while most of them exist as D-glucronic acid in heparan sulfate. The carbon atom at position 2 of the uronic acid residue partially undergoes O-sulfation. Further, sulfation occasionally occurs at another site, and such diversity in positions undergoing sulfation provides structural diversity to the molecule. Heparin undergoes sulfation at a higher degree as a total than heparan sulfate. However, heparin and heparan sulfate preparation extracted from tissues are not uniform, making it difficult to discriminate between the two families.

Heparin has been clinically used mainly as an anti-blood coagulation agent. For example, heparin is used for prevention of coagulation during ex vivo blood circulation, or in treatment or prevention of thrombotic disease including disseminated intravascular coagulation syndrome (DIC) and cardiac infarction. Heparin itself has no anti-blood coagulation activity, however, through binding to antithrombin III (ATIII) or heparin cofactor II, it inactivates various serine proteases working as a blood coagulation factor, thus inhibiting blood coagulation strongly (Bourin, M.C. et al., Biochem. J., 1993, vol.289 (Pt2), p.313-330).

Although heparin has already been used as a medicament like this, another utilization as an agent for promoting HGF production is expected (JP-A-312941/1994).

If heparin is available as an agent for promoting HGF production, therapeutic effect on various diseases may be expected through the aforementioned pharmacological actions of HGF. However, when heparin is used for promotion of HGF production, previously utilized anti-blood coagulation activity of heparin will lead to bleeding tendency, which may cause a side effect. Upon administration of heparin to a human, a dose must be carefully controlled by monitoring anti-blood coagulation activity in order to prevent bleeding during the administration. In particular, since unfractionated high-molecular-weight heparin has a strong anti-blood coagulation activity, the most careful attention should be paid upon use. Thus, anti-blood coagulation activity of heparin is a defect upon use as an agent for promoting HGF production.

In addition, heparin also has an activity to increase lipoprotein lipase (LPL) activity in plasma by releasing LPL present in blood vessel walls (Olivecrona, T. et al., Haemostasis, 1993, vol.23 (Suppl.1), p.150 to 160).

Increase in LPL activity promotes blood clarification through fat degradation, but persistent increase in LPL activity increases a free fatty acid level in blood, and may cause arrhythmia.

Therefore, a method of preparing heparin or heparan sulfate that does not have anti-blood coagulation activity and LPL releasing activity, but has only activity to promote HGF production has been keenly desired.

As a method of decreasing anti-blood coagulation activity of heparin, depolymerization of heparin to low-molecular weight fragments is known(Linhardt,R. J. et al., Semin.Thromb.Hemost., 1999, vol.25, Suppl.3, p.5 to 16). As described above, unfractionated high-molecular-weight heparin has a strong anti-blood coagulation activity and careful attention is required for its practical use. For overcoming such defect of high-molecular-weight heparin, low-molecular-weight heparin is utilized. As described above, anti-blood coagulation activity of heparin is due to the formation of heparin-ATIII complex. The heparin-ATIII complex binds to thrombin (blood coagulation factor IIa) or other coagulation factors (Xa, XIIa, XIa, IXa etc.), and inactivates them. To inhibit factor IIa, a molecular size of heparin not smaller than octadeca saccharide is necessary. Therefore, main action of high molecular weight heparin is particularly due to inactivation of factor IIa, although anti-factor Xa action is also involved. On the other hand, when heparin is depolymerized to a low-molecular size not larger than hexadecasaccharide, anti-blood coagulation activity is known to decrease, because anti-factor IIa activity seen in the polysaccharide not smaller than octadecasaccharide is reduced. (Holmer,E. et al., Haemostasis, 1986, vol.16, Suppl.2, p.1 to 7 and Lane D.A. et al. , Biochem.J. , 1984, vol.218, p.725 to 732)

In addition, in low-molecular-weight heparin, LPL releasing activity is also known to be decreased (Nasstrom,B. et al., J.Lab.Clin.Med., 2003, vol.142, p.90 to 99).

Like this, by depolymerizing heparin to a low-molecular size, it is possible to decrease anti-blood coagulation activity and LPL releasing activity, but herein, if heparin is depolymerized to a low-molecular size, whether an activity to promote HGF production is retained or not is not known. It is known that dalteparin sodium (fragmin intravenous injection; Kissei Pharmaceutical Co., Ltd.) known as low-molecular-weight heparin has HGF production promoting activity (Matsumoto,K. et al., J.Biochem., 1993, vol.114, p.820 to 826).

However, dalteparin sodium has an average molecular weight of 5000, and distribution of molecular weight ranges from 2000 to 9000. Among these, molecular size responsible for an activity to promote HGF production is unknown. In particular, whether oligosaccharides of a molecular size not larger than hexadecasaccharide (molecular weight 5000 or less) retain activity to promote HGF production or not is important, but this point was entirely unknown.

Moreover, there has not been a known method to decrease anti-blood coagulation activity and LPL releasing activity of heparin without depolymerizing to a low-molecular size while keeping the HGF production promoting activity.

### DISCLOSURE OF THE INVENTION

An object of the present invention is to provide an agent for promoting HGF production comprising a sugar chain compound such as an oligosaccharide that has a heparin structure but has no or reduced anti-blood coagulation activity and LPL releasing activity of heparin or heparin sulfate, or a salt thereof.

In order to overcome the aforementioned problems, the present inventors intensively studied on functions of heparin and heparan sulfate for promoting HGF production.

As a result, the present inventors have found that oligosaccharides of di- to hexadeca-saccharides having a structure in which uronic acid residue(s) and glucosamine residue(s) are alternately and repeatedly connected by α1,4-glycosidic linkage or β1,4-glycosidic linkage, which can be obtained by depolymerizing heparin or heparan sulfate to a low-molecular size, have an activity to promote HGF production.

In addition, the present inventors also have found that the aforementioned oligosaccharides are decreased in anti-blood coagulation activity and lipoprotein releasing activity.

It has not been known at all whether an activity to promote HGF production is retained when heparin or heparan sulfate is depolymerized to low-molecular oligosaccharides not larger than hexadecasaccharides. It is especially a surprising finding that even a disaccharide compound comprised of an uronic acid residue and a glucosamine residue that are connected by α1,4-glycosidic linkage or β1,4-glycosidic linkage has an activity to promote HGF production. The present inventors have also found that sulfation of the hydroxy group at position 6 of the glucosamine residue or the amino group at position 2 of the glucosamine residue in heparin or heparan sulfate enhances the activity for promoting HGF production. Accordingly, they have found that, when the structure of heparin or heparin sulfate is modified so that only the hydroxyl group at position 6 of the glucosamine residue is sulfated or so that only the amino group at position 2 of the glucosamine residue is sulfated, anti-blood coagulation activity and LPL releasing activity are decreased without depolymerizing heparin or heparan sulfate to a low-molecular size, while the HGF production promoting activity is retained. Based on these findings, the present inventors further extended the study and completed the present invention.

That is, the present invention relates to:
(1) an agent for promoting HGF production comprising, as an effective ingredient, a disaccharide comprised of an uronic acid residue (wherein an uronic acid means an iduronic acid or a glucuronic acid, and has the same meaning hereinafter) and a glucosamine residue that are connected by α1,4-glycosidic linkage or β1,4-glycosidic linkage, or tri- to hexadeca-saccharides having a structure in which uronic acid residue(s) and glucosamine residue(s) are alternately and repeatedly connected by α1,4-glycosidic linkage or β1,4-glycosidic linkage, wherein at least one hydroxy group of the uronic acid residue(s) and/or the glucosamine residue(s) may be sulfated, alkylated, acylated or aminated, and/or amino group at position 2 of at least one of the glucosamine residue (s) may be sulfated, alkylated or acylated, or a salt thereof,
(2) the agent for promoting HGF production according to the above (1), wherein the hydroxy group at position 2 of at least one of the uronic acid residue(s) and/or the hydroxy group at positions 3 and/or 6 of at least one of the glucosamine residue(s) may be sulfated,
(3) the agent for promoting HGF production according to the above (1) or (2), wherein the hydroxy group at position 6 and/or the amino group at position 2 of at least one of the glucosamine residue(s) is sulfated,
(4) the agent for promoting HGF production according to any one of the above (1) to (3), wherein the oligosaccharide is di- to deca-saccharide,
(5) the agent for promoting HGF production according to any one of the above (1) to (4), wherein the oligosaccharide is a degradation product prepared from high-molecular-weight heparin or high-molecular-weight heparan sulfate by digestion with heparinase or heparitinase,
(6) the agent for promoting HGF production according to any one of the above (1) to (4), wherein the oligosaccharide is a degradation product prepared from high-molecular-weight heparin or high-molecular-weight heparan sulfate by digestion by any one of nitrous acid degradation, hydrogen peroxide degradation or β-elimination,
(7) the agent for promoting HGF production according to the above (1), wherein the oligosaccharide is any one of compounds represented by the following (a) to (h);
   (a) formula (I): 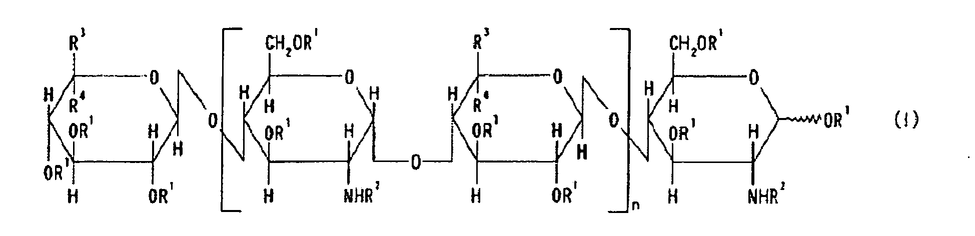 wherein R¹ represents hydrogen, sulfate group, alkyl, acyl, or optionally substituted amino group, R² represents hydrogen, sulfate group, alkyl or acyl group, R³ and R⁴ are different from each other and represent hydrogen or optionally substituted carboxyl group, and n represents 0 to 7,
   (b) formula (II): 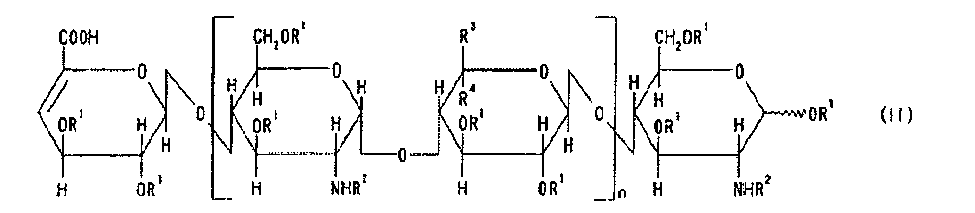 wherein all the symbols are respectively the same as defined above,
   (c) formula (III): 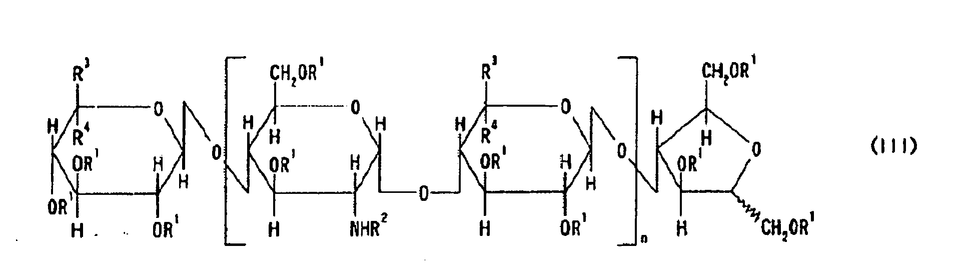 wherein all the symbols are respectively the same as defined above,
   (d) formula (IV): 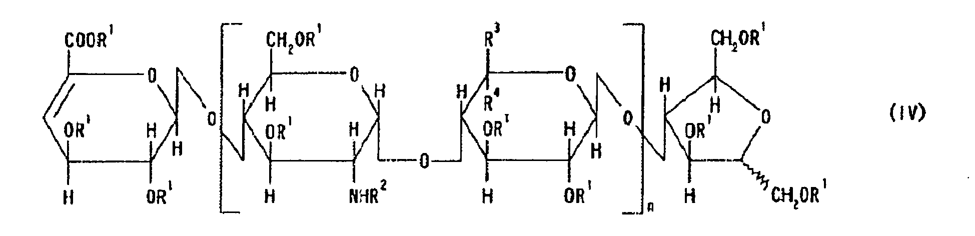 wherein all the symbols are respectively the same as defined above,
   (e) formula (V): 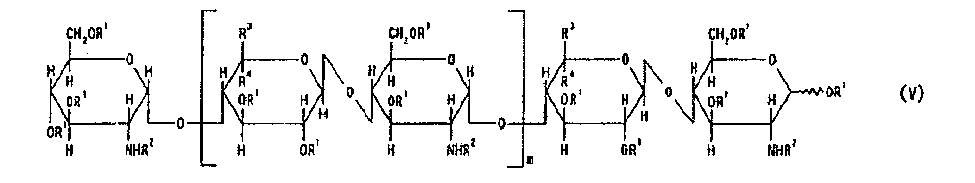 wherein m represents 0 to 6, and R¹, R², R³ and R⁴ are respectively the same as defined above,
   (f) formula (VI): 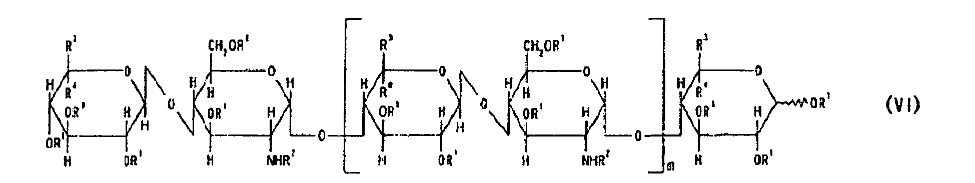 wherein m represents 0 to 6, and R¹, R², R³ and R⁴ are respectively the same as defined above,
   (g) formula (VII) 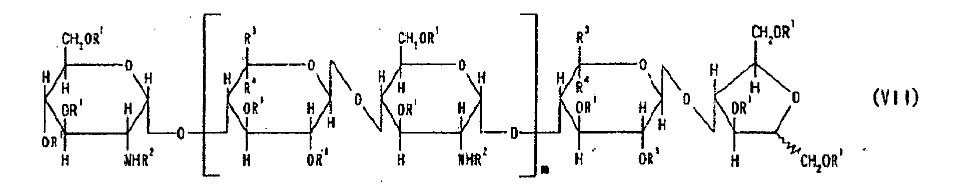 wherein m represents 0 to 6, and R¹, R², R³ and R⁴ are respectively the same as defined above, and
   (h) formula (VIII) 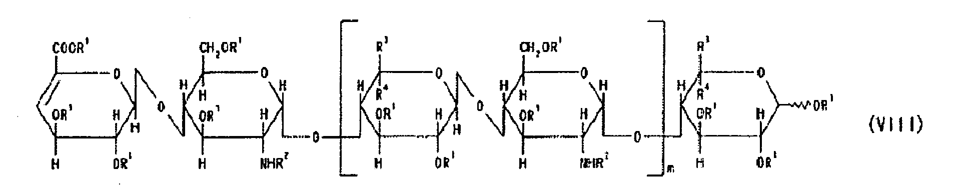
   wherein m represents 0 to 6, and R¹, R², R³ and R⁴ are respectively the same as defined above,
(8) an agent for promoting HGF production comprising, as an effective ingredient, a sugar chain compound having a structure in which uronic acid residue (s) and glucosamine residue (s) are alternately and repeatedly connected by α1,4-glycosidic linkage or β1,4-glycosidic linkage, wherein the hydroxy group at position 6 of at least one of the glucosamine residue(s) is sulfated, or a salt thereof,
(9) an agent for promoting HGF production comprising, as an effective ingredient, a sugar chain compound having a structure in which uronic acid residue(s) and glucosamine residue(s) are alternately and repeatedly connected by α1,4-glycosidic linkage or β1,4-glycosidic linkage, wherein the amino group at position 2 of at least one of the glucosamine residue(s) is sulfated, or a salt thereof,
(10) an agent for promoting HGF production comprising, as an effective ingredient, a disaccharide compound comprised of an uronic acid residue and a glucosamine residue wherein the hydroxy group at position 6 of the glucosamine residue and/or the amino group at position 2 of the glucosamine residue are/is sulfated are connected by α1,4-glycosidic linkage or β1,4-glycosidic linkage, or a salt thereof, and
(11) the agent for promoting HGF production according to any one of the above (1) to (10), wherein the sugar chain compound or a salt thereof has no or reduced anti-blood coagulation activity and/or lipoprotein lipase releasing activity.
   Also, the present invention relates to:
(12) a method of promoting HGF production characterized by administering to a mammal an effective amount of a disaccharide composed of an uronic acid residue (wherein an uronic acid means an iduronic acid or a glucuronic acid, and has the same meaning hereinafter) and a glucosamine residue that are connected by α1,4-glycosidic linkage or β1,4-glycosidic linkage, or tri- to hexadeca-saccharides having a structure in which uronic acid residue(s) and glucosamine residue(s) are alternately and repeatedly connected by α1,4-glycosidic linkage or β1,4-glycosidic linkage, wherein at least one hydroxy group of the uronic acid residue(s) and/or the glucosamine residue(s) may be sulfated, alkylated, acylated or aminated, and/or the amino group at position 2 of at least one of the glucosamine residue(s) may be sulfated, alkylated or acylated, or a salt,
(13) the method of promoting HGF production according to the above (12), wherein the hydroxy group at position 2 of at least one of the uronic acid residue(s) and/or the hydroxy group at positions 3 and/or 6 of at least one of the glucosamine residue(s) may be sulfated,
(14) the method of promoting HGF production according to the above (12), wherein the hydroxy group at position 6 and/or the amino group at position 2 of at least one of the glucosamine residue(s) is sulfated,
(15) the method of promoting HGF production according to the above (12), wherein the oligosaccharide is di- to deca-saccharide,
(16) the method of promoting HGF production according to the above (12), wherein the oligosaccharide is a degradation product prepared from high-molecular-weight heparin or high-molecular-weight heparan sulfate by digestion with heparinase or heparitinase,
(17) the method of promoting HGF production according to the above (12), wherein the oligosaccharide is a degradation product prepared from high-molecular-weight heparin or high-molecular-weight heparan sulfate by any one of nitrous acid degradation, hydrogen peroxide degradation or β-elimination,
(18) the method of promoting HGF production according to the above (12), wherein the oligosaccharide is any one of compounds represented by the following (a) to (h);
   (a) formula (I): 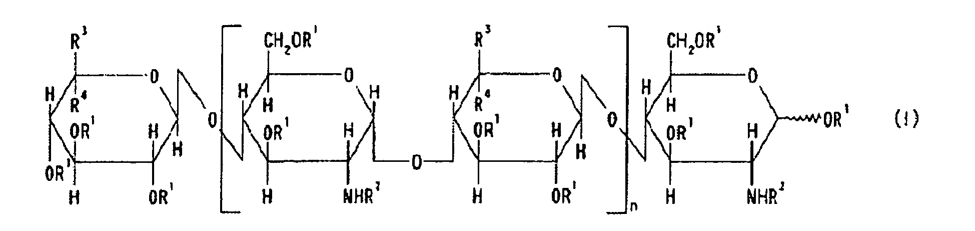 wherein R¹ represents hydrogen, sulfate group, alkyl, acyl, or optionally substituted amino group, R² represents hydrogen, sulfate group, alkyl or acyl group, R³ and R⁴ are different from each other and represent hydrogen or optionally substituted carboxyl group, and n represents 0 to 7,
   (b) formula (II): 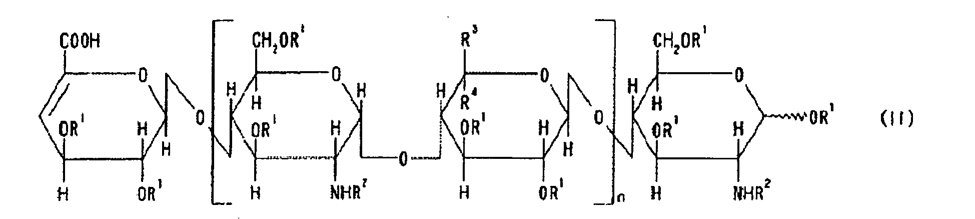 wherein all the symbols are respectively the same as defined above,
   (c) formula (III): 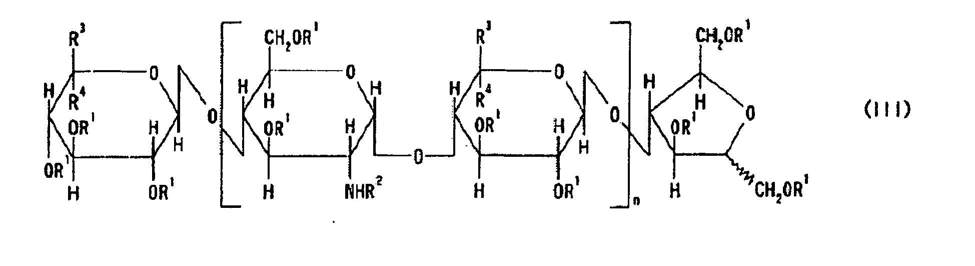 wherein all the symbols are respectively the same as defined above,
   (d) formula (IV): 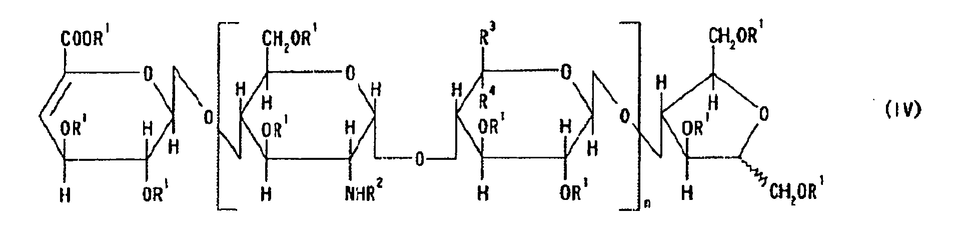 wherein all the symbols are respectively the same as defined above,
   (e) formula (V): 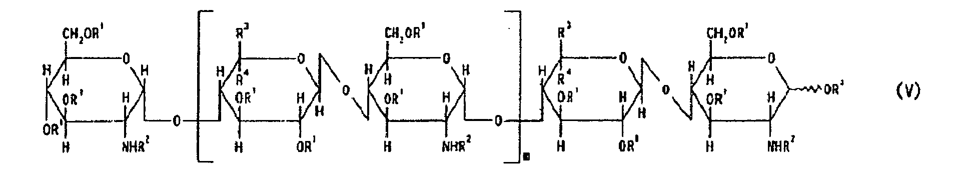 wherein m represents 0 to 6, and R¹, R², R³ and R⁴ are respectively the same as defined above,
   (f) formula (VI): 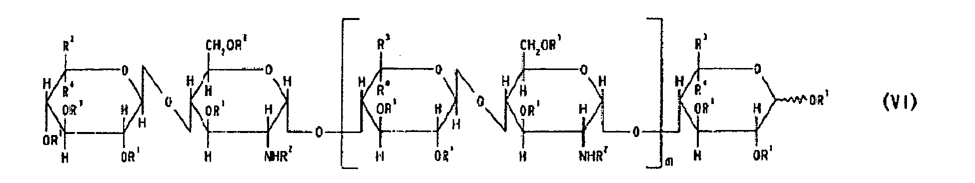 wherein m represents 0 to 6, and R¹, R², R³ and R⁴ are respectively the same as defined above,
   (g) formula (VII) 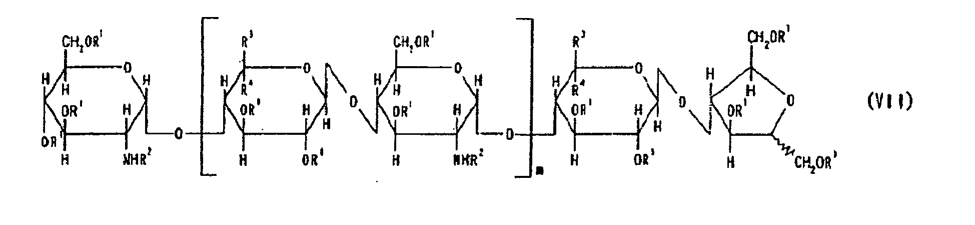 wherein m represents 0 to 6, and R¹, R², R³ and R⁴ are respectively the same as defined above, and
   (h) formula (VIII) 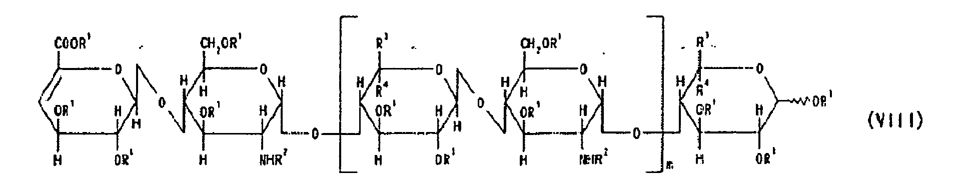
   wherein m represents 0 to 6, and R¹, R², R³ and R⁴ are respectively the same as defined above,
(19) a method of promoting HGF production characterized by administering to a mammal an effective amount of a sugar chain compound having a structure in which uronic acid residue (s) and glucosamine residue(s) are alternately and repeatedly connected by α1,4-glycosidic linkage or β1,4-glycosidic linkage, wherein the hydroxy group at position 6 of at least one of the glucosamine residue(s) is sulfated, or a salt thereof,
(20) a method of promoting HGF production characterized by administering to a mammal an effective amount of a sugar chain compound having a structure in which uronic acid residue (s) and glucosamine residue(s) are alternately and repeatedly connected by α1,4-glycosidic linkage or β1,4-glycosidic linkage, wherein the amino group at position 2 of at least one of the glucosamine residue(s) is sulfated, or a salt thereof,
(21) a method of promoting HGF production characterized by administering to a mammal an effective amount of a disaccharide compound comprised of an uronic acid residue and a glucosamine residue in which the hydroxy group at position 6 and/or the amino group at position 2 of the glucosamine residue are/is sulfated are connected by α1,4-glycosidic linkage or β1,4-glycosidic linkage, or a salt thereof, and
(22) the method of promoting HGF production according to any one of the above (12) to (21), wherein the sugar chain compound or a salt thereof has no or reduced anti-blood coagulation activity and/or lipoprotein lipase releasing activity.
   Furthermore, the present invention relates to:
(23) use of a disaccharide composed of an uronic acid residue(wherein an uronic acid means an iduronic acid or a glucuronic acid, and has the same meaning hereinafter) and a glucosamine residue that are connected by α1,4-glycosidic linkage or β1,4-glycosidic linkage, or tri- to hexadeca-saccharides having a structure in which uronic acid residue(s) and glucosamine residue(s) are alternately and repeatedly connected by α1,4-glycosidic linkage or β1,4-glycosidic linkage, wherein at least one hydroxy group of the uronic acid residue(s) and/or the glucosamine residue(s) may be sulfated, alkylated, acylated or aminated, and/or the amino group at position 2 of at least one of the glucosamine residue(s) may be sulfated, alkylated or acylated, or a salt thereof, for the production of a medicament for promoting HGF production,
(24) the use according to the above (23), wherein the hydroxy group at position 2 of at least one of the uronic acid residue(s) and/or the hydroxy group at positions 3 and/or 6 of at least one of the glucosamine residue(s) may be sulfated,
(25) the use according to the above (23), wherein the hydroxy group at position 6 and/or the amino group at position 2 of at least one of the glucosamine residue(s) is sulfated,
(26) the use according to the above (23), wherein the oligosaccharide is di- to deca-saccharide,
(27) the use according to the above (23), wherein the oligosaccharide is a degradation product prepared from high-molecular-weight heparin or high-molecular-weight heparan sulfate by digestion with heparinase or heparitinase,
(28) the use according to the above (23), wherein the oligosaccharide is a degradation product prepared from high-molecular-weight heparin or high-molecular-weight heparan sulfate by any one of nitrous acid degradation, hydrogen peroxide degradation or β-elimination,
(29) the use according to the above (23) wherein the oligosaccharide is any one of compounds represented by the following (a) to (h);
   (a) formula (I): 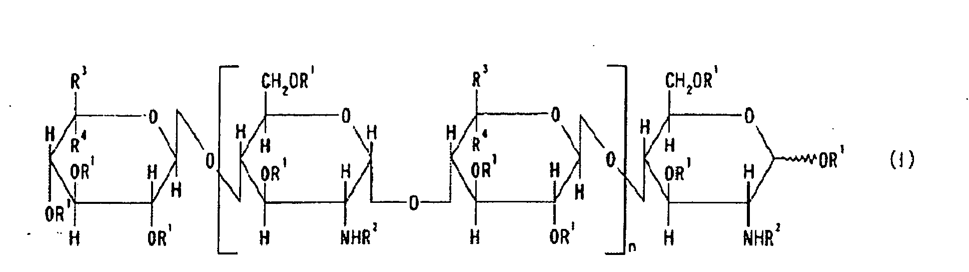 wherein R¹ represents hydrogen, sulfate group, alkyl, acyl, or optionally substituted amino group, R² represents hydrogen, sulfate group, alkyl or acyl group, R³ and R⁴ are different from each other and represent hydrogen or optionally substituted carboxyl group, and n represents 0 to 7,
   (b) formula (II): 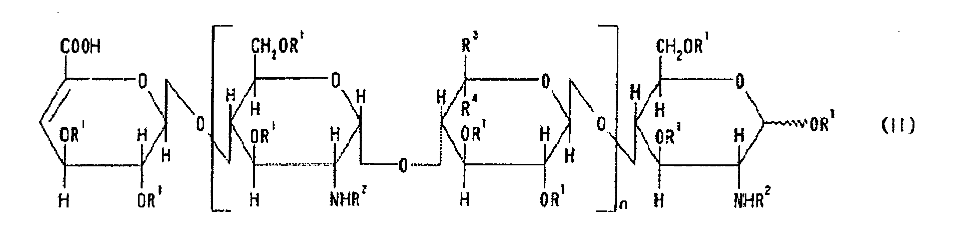 wherein all the symbols are respectively the same as defined above,
   (c) formula (III): 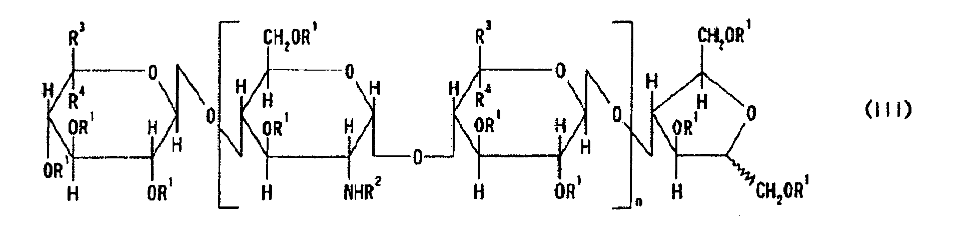 wherein all the symbols are respectively the same as defined above,
   (d) formula (IV): 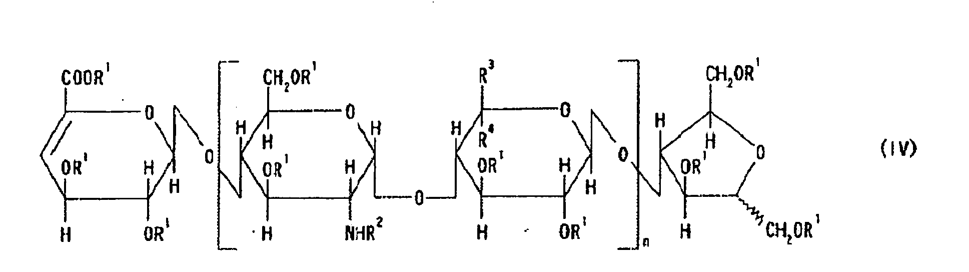 wherein all the symbols are respectively the same as defined above,
   (e) formula (V): 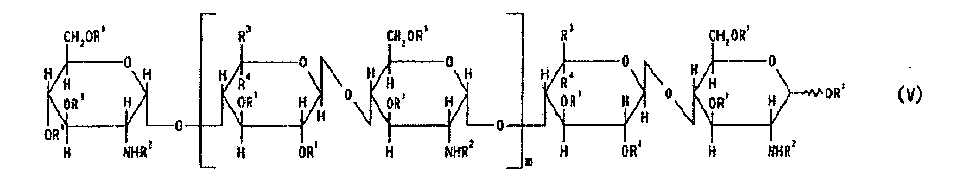 wherein m represents 0 to 6, and R¹, R², R³ and R⁴ are respectively the same as defined above,
   (f) formula (VI): 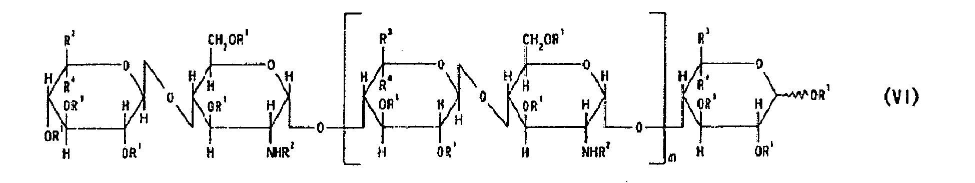 wherein m represents 0 to 6, and R¹, R², R³ and R⁴ are respectively the same as defined above,
   (g) formula (VII) 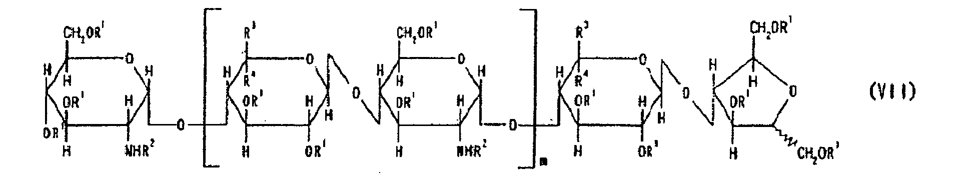 wherein m represents 0 to 6, and R¹, R², R³ and R⁴ are respectively the same as defined above, and
   (h) formula (VIII) 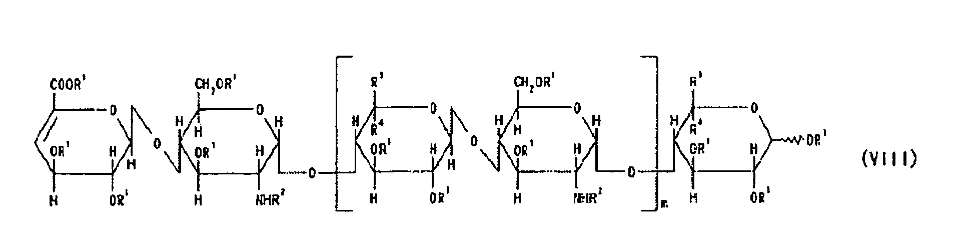
   wherein m represents 0 to 6, and R¹, R², R³ and R⁴ are respectively the same as defined above,
(30) use of a sugar chain compound having a structure in which uronic acid residue(s) and glucosamine residue(s) are alternately and repeatedly connected by α1,4-glycosidic linkage or β1,4-glycosidic linkage, wherein the hydroxy group at position 6 of at least one of the glucosamine residue(s) is sulfated, or a salt thereof, for the production of a medicament for promoting HGF production,
(31) use of a sugar chain compound having a structure in which uronic acid residue(s) and glucosamine residue(s) are alternately and repeatedly connected by α1,4-glycosidic linkage or β1,4-glycosidic linkage, wherein at least one amino group at position 2 of the glucosamine residue(s) is sulfated, or a salt thereof, for the production of a medicament for promoting HGF production,
(32) use of a disaccharide compound comprised of an uronic acid residue and a glucosamine residue wherein the hydroxy group at position 6 and/or the amino group at position 2 of the glucosamine residue are/is sulfated are connected by α1,4-glycosidic linkage or β1,4-glycosidic linkage, or a salt thereof, for the production of a medicament for promoting HGF production, and
(33) the use according to any one of the above (23) to (32), wherein the sugar chain compound or a salt thereof has no or reduced anti-blood coagulation activity and/or lipoprotein lipase releasing activity.

Although sugar chain compounds such as oligosaccharides used in the agent of the present invention for promoting HGF production have a heparin structure constructed by repeated disaccharides composed of an uronic acid residue and a glucosamine residue that are connected by α1,4-glycosidic linkage or β1,4-glycosidic linkage, they can promote HGF production with no or less side effect of bleeding tendency. The sugar chain compound has an effect for facilitating healing from damages of tissues and organs of a living body. More specifically, the sugar chain compound is useful in the treatment of liver disease, kidney disease, skin disease, blood disease, eye disease, lung disease, stomach or duodenum disease, cancer and associated diseases thereof, bone disease, and diseases of the central nervous system.

### BRIEF DESCRIPTION OF THE DRAWINGS

Fig. 1 shows a separation pattern of depolymerized heparin products on a gel filtration column.
Fig. 2 shows activity of heparin fragments to promote HGF production.
Fig. 3 shows a separation pattern of heparin decasaccharide fragments on an anion-exchange column.
Fig. 4 shows HGF production-promoting activity of the heparin decasaccharide fragments that are separated by anion-exchange chromatography.
Fig. 5 shows results of MALDI -TOF/MS analysis of the heparin decasaccharide fragments that are separated by anion-exchange chromatography.
Fig. 6 shows HGF production-promoting activity of various disaccharides that are sulfated at different positions.
Fig. 7 shows anti-blood coagulation activity of heparin fragments, which were assessed by activated partial thromboplastin time (hereinafter abbreviated as APTT).
Fig. 8 shows LPL releasing activity of heparin fragments.
Fig. 9 shows anti-blood coagulation activity of various disaccharide compounds sulfated at different positions, which were assessed by APTT.
Fig 10 shows lipoproteinlipase (hereinafter abbreviated as LPL) releasing activity of various disaccharide compounds sulfated at different positions.
Fig. 11 shows HGF production-promoting activity of modified heparin in which only the amino group at position 2 of the glucosamine residue is sulfated.
Fig. 12 shows anti-blood coagulation activity of modified haparin in which only the amino group at position 2 of the glucosamine residue is sulfated.

### BEST MODE FOR CARRYING OUT THE INVENTION

In the present invention, a heparin structure refers to a structure in which uronic acid residues and glucosamine residues are alternately and repeatedly connected by α1,4-glycosidic linkage or β1,4-glycosidic linkage.

An uronic acid residue may be either an L-iduronic acid residue (hereinafter, abbreviated as IdoA) or a D-glucronic acid residue (hereinafter, abbreviated as GlcA). Uronic acid residues may be present as only IdoA or only GlcA, or as both IdoA and GlcA, and proportion of IdoA and GlcA is not particularly limited.

Also, an oligosaccharide in the present invention refers to a heterooligosaccharide comprised of two different saccharides of an uronic acid and a glucosamine, specifically a disaccharide compound comprised of an uronic acid residue and a glucosamine residue that are connected by α1,4-glycosidic linkage or β1,4-glycosidic linkage; a trisaccharide compound comprised of an uronic acid residue or a glucosamine residue and the said disaccharide compound that are connected by α1,4-glycosidic linkage or β1,4-glycosidic linkage; or a sugar chain compound of tetra- to hexadeca-saccharides having a structure in which an uronic acid residue and a glucosamine residue are alternately and repeatedly connected by α1,4-glycosidic linkage or β1,4-glycosidic linkage.

Further, in the present invention, alkylation refers to substitution of the hydrogen of a hydroxy group of an uronic acid residue or/and a hydroxy group or/and an amino group of a glucosamine residue with alkyl, preferably lower alkyl group.

Acylation refers to substitution of the hydrogen of a hydroxy group of an uronic acid residue or/and a hydroxy group or/and an amino group of a glucosamine residue with an acyl group represented by the formula - (C=O)-R⁵, -(C=O)-OR⁵, -(C=O)-NR⁵R⁶,(C=S)-NHR⁵, -SO-R⁵, -SO₂-R⁵ or -SO₂-NHR (wherein R⁵ and R⁶ represent a hydrogen atom or a hydrocarbon group). In the aforementioned formulas, examples of the hydrocarbon group represented by R⁵ and R⁶ include a chain hydrocarbon group (e.g. alkyl, alkenyl, alkynyl, etc.). As alkyl, lower alkyl is preferable. As alkenyl, for example, C₂₋₆alkenyl (e.g. vinyl, allyl, isopropenyl, 1-butenyl, 2-butenyl, 3-butenyl, 2-methyl-2-propenyl, 1-methyl-2-propenyl, 2-methyl-1-propenyl, etc.) is preferable. As alkynyl, for example, C₂₋₆alkynyl (e.g. ethynyl, propargyl, 1-butynyl, 2-butynyl, 3-butynyl, 1-hexynyl, etc.) is preferable.

Amination refers to substitution of the hydrogen of a hydroxy group of an uronic acid residue or/and a hydroxy group of a glucosamine residue with an amino group. The amino group may be further substituted with a sulfate group, alkyl group or acyl group.

In the present invention, "lower alkyl" refers to alkyl with 1 to 6 carbon atoms, such as methyl, ethyl, propyl, isopropyl, butyl, isobutyl, sec-butyl, tert-butyl, pentyl, hexyl, and the like.

In uronic acid residues constituting a sugar chain compound such as oligosaccharides used in the agent of the present invention, at least one hydroxy group at positions 2 and 3 may be sulfated, alkylated, acylated or aminated. Preferable is sulfation, and particularly preferable is sulfation of hydroxy group at position 2.

In glucosamine residues constituting a sugar chain compound such as oligosaccharides used in the agent of the present invention, the amino group at position 2 may be sulfated, alkylated or acylated, preferably sulfated or acylated, and more preferably sulfated. In the acylation, acylation with -(C=O)-R⁵ is more preferable and, inter alia, acylation with acetyl group is particularly preferable.

In addition, at least one of the hydroxy groups at positions 3 and 6 of the glucosamine residue may be sulfated, alkylated or acylated, preferably sulfated. Sulfation of hydroxy group at position 6 is particularly preferable.

With the increase in the number of repetition of disaccharides composed of an uronic acid residue and a glucosamine residue, the activity of oligosaccharides of the present invention for promoting HGF production is increased. On the other hand, anti-blood coagulation activity and LPL releasing activity are decreased with the decrease in number of repetition of disaccharides. Considering these facts, a di-to hexadeca-saccharide, preferably a di- to deca-saccharide, is used in the present invention from the viewpoint that they can promote HGF production with no or less side effect of anti-blood coagulation activity and LPL releasing activity.

With regard to the di- to hexadeca-saccharides used in the agent of the present invention for promoting HGF production, HGF production-promoting activity is enhanced with the increase in the degree of sulfation per molecule. The preferable degree of the sulfation is about 1 to 3 sulfate group(s) per two sugar residues.

Although there is no limitation in the positions of the sulfate groups in the di- to hexadeca-saccharides, it is preferable that the hydroxy group at position 6 and/or the amino group at position 2 of at least one of the glucosamine residue(s) in the sugar chain compound are/is sulfated. When the sugar chain compound is sulfated like this, sulfation at other positions in the sugar chain compound may not be necessary. In the case of disaccharide compounds, it is preferable that at least either one of the hydroxy group at position 6 and/or the amino group at position 2 of the glucosamine residue are/is sulfated.

Preferable examples of an oligosaccharide used in the agent of the present invention for promoting HGF production include compounds shown below and a salt thereof.
(a) formula (I): 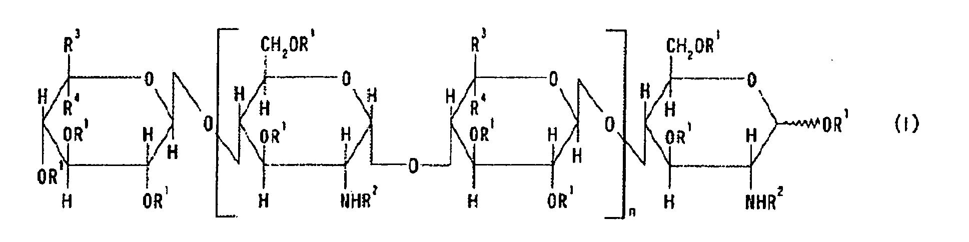 (wherein R¹ represents hydrogen, sulfate group, alkyl, acyl or optionally substituted amino group, R² represents hydrogen, sulfate group, alkyl or acyl group, R³ and R⁴ are different from each other, and represent hydrogen or optionally substituted carboxyl group, and n represents 0 to 7)
(b) formula (II): 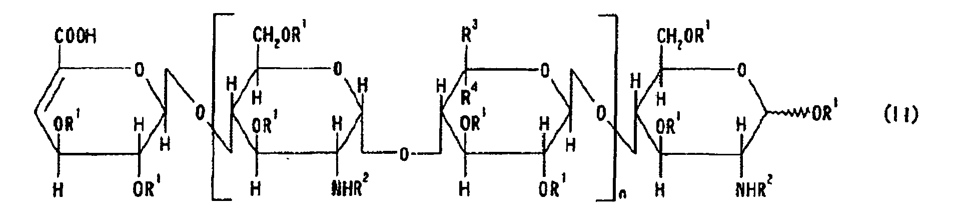 (wherein all the symbols are respectively the same as defined above)
(c) formula(III) 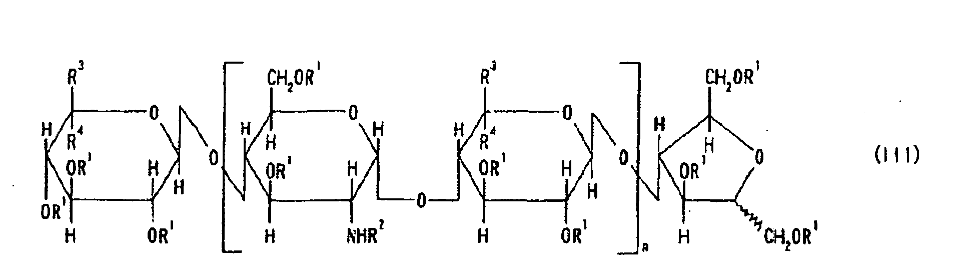 (wherein all the symbols are respectively the same as defined above)
(d) formula (IV): 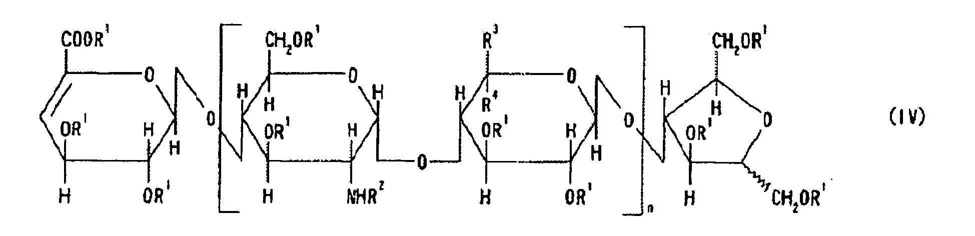 (wherein all the symbols are respectively the same as defined above)
(e) formula (V): 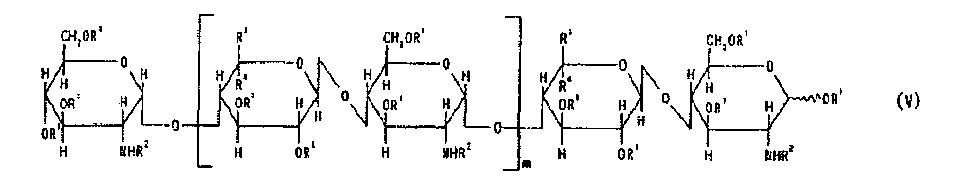 (wherein m represents 0 to 6, and R¹, R², R³ and R⁴ are respectively the same as defined above)
(f) formula (VI): 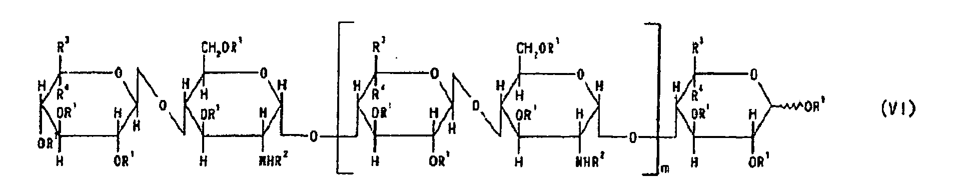 (wherein m represents 0 to 6, and R¹, R², R³ and R⁴ are respectively the same as defined above)
(g) formula (VII): 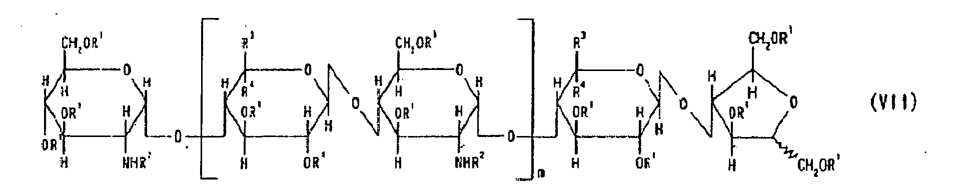 (wherein m represents 0 to 6, and R¹, R², R³ and R⁴ are respectively the same as defined above)
(h) formula (VIII): 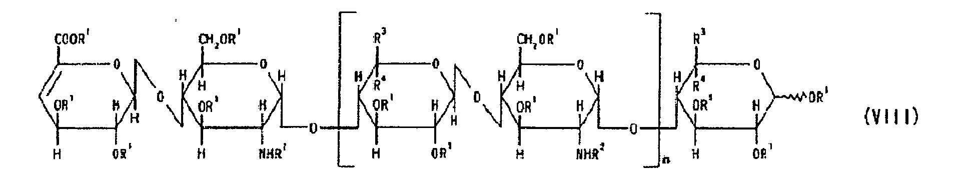 (wherein m represents 0 to 6, and R^{1,} R², R³ and R⁴ are respectively the same as defined above)
(i) a disaccharide compound comprised of an uronic acid residue and a glucosamine residue in which only the hydroxy group at position 6 is sulfated are connected by α1,4-glycosidic linkage or β1,4-glycosidic linkage
(j) a disaccharide compound comprised of an uronic acid residue and a glucosamine residue in which only the amino group at position 2 is sulfated are connected by α1,4-glycosidic linkage or β1,4-glycosidic linkage.

As alkyl groups represented by R¹ and R² shown in the aforementioned formulas (I) to (VIII), lower alkyl groups with 1 to 6 carbon atoms are preferable, and examples thereof include methyl, ethyl, propyl, isopropyl, butyl, isobutyl, sec-butyl, tert-butyl, pentyl, hexyl, and the like. Examples of acyl groups represented by R¹ and R² include those represented by the formula -(C=O)-R³, -(C=O)-OR³, -(C=O)-NR³R⁴, -(C=S)-NHR³, -SO-R⁵, -SO₂-R⁵ or -SO₂-NHR³ (wherein R³ and R⁴ each represents a hydrogen atom or a hydrocarbon group, R⁴ represents a hydrogen atom, or lower alkyl group with 1 to 6 carbon atoms, and R⁵ represents a hydrocarbon group optionally having a substituent). Among the acyl groups, -(C=O)-R³ is preferable, and acetyl is particularly preferable. In the aforementioned formulas, examples of hydrocarbon groups represented by R³ and R⁵ include chain hydrocarbon groups (e.g. alkyl, alkenyl, alkynyl, etc.). As the alkyl groups, lower alkyl groups having 1 to 6 carbon atoms (e.g. methyl, ethyl, propyl, isopropyl, butyl, isobutyl, sec-butyl, tert-butyl, pentyl, hexyl, etc.) are preferable. As the alkenyl groups, for example, C₂₋₆ alkenyl groups (e.g. vinyl, allyl, isopropenyl, 1-butenyl, 2-butenyl, 3-butenyl, 2-methyl-2-propenyl, 1-methyl-2-propenyl, 2-methyl-1-propenyl, etc.) are preferable. As the alkynyl groups, for example, C₂₋₆alkynyl groups (e.g. ethynyl, propargyl, 1-butynyl, 2-butynyl, 3-butynyl, 1-hexynyl, etc.) are preferable.

Examples of a substituent which may substitute an amino group used in R² shown in the formulas (I) to (VIII) include a sulfate group, and alkyl and acyl groups, preferably a sulfate group and an acyl group, and more preferably a sulfate group. As the acyl group, -(C=O)-R⁵ is more preferable, and acetyl is particularly preferable. The alkyl and the acyl groups mean the same substituent as described for alkyl and acyl groups used in R¹ and R².

As a preferable combination of R¹ and R², R¹ is hydrogen or a sulfate group, and R² is hydrogen, a sulfate group or an acyl group. In this case, as the acyl group, -(C=O)-R³ is preferable, and acetyl is particularly preferable.

And, n is 0 to 7, preferably 0 to 4, and m is 0 to 6, preferably O to 4.

As salts of the aforementioned oligosaccharide such as the compounds represented by the formulas (I) to (VIII), included are alkali metal salts such as lithium salt, sodium salt and potassium salt, salts of alkaline earth metal salts such as calcium salt, and acid salts such as inorganic acid salts (e.g. hydrochloride, sulfate, hydrobromide, phosphate, etc.), and organic acid salts (e.g. acetate, trifluoroacetate, succinate, maleate, fumarate, propionate, citrate, tartarate, lactate, oxalate, methanesulfonate, p-toluenesulfonate, etc.).

The oligosaccharides or a salt thereof used in the agent of the present invention for promoting HGF production can usually be prepared from heparin or heparan sulfate. Heparin may be either high-molecular-weight heparin or low-molecular-weight heparin, and heparan sulfate may be either high-molecular-weight heparan sulfate or low-molecular-weight heparan sulfate. The oligosaccharides used in the present invention or a salt thereof can be obtained by fragmenting high-molecular-weight heparin or high-molecular-weight heparan sulfate. The starting high-molecular-weight heparin or high-molecular-weight heparan sulfate may be those obtained by extraction from biomaterials, or those or analogs thereof prepared by chemical synthesis.

To produce high-molecular-weight heparin or high-molecular-weight heparan sulfate for a raw material by extraction from biomaterials, various mammal tissues (e.g. spleen, lung. muscle, etc. ) including human tissues can be used, and, generally, those derived from porcine or ovine intestinal mucosa origin or bovine lung origin are used. A preferable source for heparin is porcine intestinal mucosa. Generally, heparin can be prepared from a selected tissue source as follows. A tissue material is autolyzed, from which tissue extract is prepared with an alkali, and proteins are coagulated, and a heparin-protein complex is precipitated from the supernatant by oxidation. The complex is recovered by re-precipitation using a polar non-aqueous solvent such as ethanol, acetone and a mixture thereof, and a fat is removed by extraction with an organic solvent such as ethanol. Then the complex is treated with a protease such as trypsin to remove proteins. A method of preparing heparin is described, for example, in The Japanese Pharmacopoeia, Fourteenth Edition, or an article of Charles, A. F. et al. (Biochem. J., 1936, vol. 30, p. 1927-1933) , and amodified method is also known, as disclosed in Chemistry and Biology of Heparin (1981)(edited by Lundblad,R.L. et al., Elsevier Publishers, North Holland, New York). Generally, high-molecular-weight heparin or high-molecular-weight heparan sulfate can be obtained from a tissue by a conventional method by which heparin is prepared as an anti-blood coagulant agent (Linhardt,R.J. et al, Semin.Thromb.Hemost., 1999, vol.25, Suppl.3, p.5 to 16).

As described above, high-molecular-weight heparin or high-molecular-weight heparan sulfate obtained by extraction from a biomaterial is a polysaccharide composed of the repetition of, as a constitution unit, a disaccharide comprising uronic acid (IdoA or GlcA) and glucosamine. GlcA and IdoA are present in both of heparin and heparan sulfate. IdoA as uronic acid is more abundant in heparin, while GlcA is enriched in heparan sulfate. GlcA and IdoA are present as a mixture because partial epimerization at the carbon atom of position 5 of GlcA residues takes place, and thereby GlcA is converted into IdoA. In proportion as heparan sulfate becomes more heparin-like, a ratio of IdoA/GlcA is increased.

Alternatively, high-molecular-weight heparin or high-molecular-weight heparan sulfate used for a raw material may be chemically synthesized ones or their analogs. In this case, their composition may not equal to the aforementioned natural one, however generally termed heparin or heparan sulfate or their analogs can be used as a raw material for an oligosaccharide or a salt thereof used in the agent of the present invention for promoting HGF production.

Further, in the present invention, low-molecular-weight heparin or low-molecular-weight heparan sulfate having a molecular weight of 3000 to 6000 may be used as a raw material. In low-molecular-weight heparin having a molecular weight of 3000 to 6000, anti-factor Xa activity is strong while its anti-thrombin activity is weak, and such low-molecular-weight heparin is clinically applied due to its little risk of bleeding. The low-molecular-weight heparin having a molecular weight of 3000 to 6000, is weak in platelet aggregation action, and is also weak in action to release LPL and the like from a blood vessel wall. In addition, low-molecular weight heparin shows good absorption via subcutaneous injection. From these points, utilization of the low-molecular-weight heparin has been developed in recent years. An oligosaccharide or a salt thereof used in the agent of the present invention for promoting HGF production may be further lower-molecular size than low-molecular-weight heparin or low-molecular-weight heparan sulfate having a molecular weight of 3000 to 6000. Therefore, even those which have been generally called low-molecular-weight heparin or low-molecular-weight heparan sulfate can be used as a raw material for an oligosaccharide or a salt thereof in the agent of the present invention for promoting HGF production.

In order to fragment high-molecular-weight heparin or high-molecular-weight heparan sulfate or low-molecular-weight heparin having a molecular weight of 3000 to 6000, methods of enzymatically or chemically cleaving the glycosidic linkages are used. For enzymatic digestion, enzymes such as heparinase and heparitinase are used. For example, an oligosaccharide of the present invention can be prepared by degrading high-molecular-weight heparin or high-molecular-weight heparan sulfate with a heparin-degrading enzyme that depolymerizes heparin to low-molecular fragments, such as heparitinase I, heparitinase II, and heparinase. Usually, the enzymatic reaction is performed at about 20 to 45°C, preferably about 30 to 40°C, for 3 hours or longer, preferably 6 hours or longer, more preferably about 8 to 12 hours, with an enzyme of about 0.02 to 2.0 U, preferably about 0.1 to 1.0 U per 100 mg of starting high-molecular-weight heparin or high-molecular-weight heparan sulfate. However, reaction conditions are not limited to the aforementioned conditions, but can be appropriately changed. For chemical degradation, the known method such as a nitrous acid degradation method, a hydrogen peroxide degradation method, and a β-elimination method can be used ( Linhardt, R.J., et al, Semin. Thromb. Hemost., 1999, vol.25, Suppl.3, p5 to 16).

In order to obtain and purify an oligosaccharide or a salt thereof used in the present invention from a mixture of fragmented high-molecular-weight heparin or high-molecular-weight heparan sulfate, gel filtration chromatography and ion exchange chromatography can be performed alone or in combination thereof. For example, when a Superdex 30pg column (column 1. 6 cm × 60 cm, flow rate 0.4 ml/min.) manufactured by Amersham Bioscience is used in gel filtration chromatography, a hexadeca-saccharide fraction can be eluted at about 135 to 138 minutes, a tetradeca-saccharide fraction at about 140 to 144 minutes, a dodeca-saccharide fraction at about 146 to 151 minutes, a deca-saccharide fraction at about 154 to 161 minuets, an octa-saccharide fraction at about 163 to 172 minutes, a hexa-saccharide fraction at about 175 to 188 minutes, a tetra-saccharide fraction at about 190 to 209 minutes, and a di-saccharide fraction at about 216 to 249 minutes. A fraction comprising a substance having a uniform structure may be obtained by ion exchange chromatography by further separation of oligosaccharides obtained in the aforementioned fractionation. When ion exchange chromatography is used, it is preferable to desalt the resulting fraction by a conventional method, because salt concentration of the fraction frequently becomes high.

Incorporation of a column chromatography step using a resin coupled with antithrombin III into a purification process is preferable. Because, when the step is incorporated, oligosaccharides without anti-factor Xa activity can be obtained by recovering fractions not adsorbed onto the column. Thus, it is possible to obtain oligosaccharides from which an anti-blood coagulation activity is completely removed. Because the oligosaccharides flowing through the antithrombin III column retain an activity to promote HGF production, they can be applied to the present invention.

Further, the resulting oligosaccharides obtained by the above method may be sulfated, alkylated, acylated or aminated by known methods.

An oligosaccharide or a salt thereof used in the agent of the present invention for promoting HGF production may be artificially synthesized by chemical synthesis. For example, it can be prepared by synthesizing a disaccharide unit from IdoA or GlcA or a derivative thereof, and glucosamine or a derivative thereof, and polymerizing such disaccharide unit (Karst, N.A. andLinhardt, R.J., Curr. Med. Chem., 2003, vol. 10, p.1993-2031, Lee, J.C., J.Am.Chem.Soc., 2004, vol.126, p.476-7). Alternatively, chemoenzymatic synthesis combining chemical and enzymatic reactions may be performed to obtain such oligosaccharides (Kuberan, B.et al., J.Biol.Chem., 2003, vol.278, p.52613-21). Those chemical synthesis and chemoenzymatic synthesis mentioned above are particularly effective for selectively sulfating the hydroxy group at position 6 and/or the amino group at position 2 of a glucosamine residue.

Moreover, the agent of the present invention for promoting HGF production is not limited to oligosaccharides obtained as mentioned above.

A sugar chain compound not smaller than heptadecasaccharides, having a structure in which uronic acid residue(s) and glucosamine residue(s) are alternately and repeatedly connected by α1,4-glycosidic linkage or β1,4-glycosidic linkage, wherein the hydroxy group at position 6 of at least one of the glucosamine residue(s) or the amino group at position 2 of at least one of the glucosamine residue (s) is sulfated, and other hydroxy group(s) or other amino group(s) are not sulfated, can be used for the agent for promoting HGF production, since it has a lower anti-blood coagulation activity and a lower LPL releasing activity compared with heparin, while it retains an activity to promote HGF production. Such sugar chain compounds can be prepared by, for example, chemical modification of the aforementioned heparin or heparan sulfate.

The sugar chain length of the sugar chain compound not smaller than heptadecasaccharides is usually not larger than 500 saccharides, preferably not larger than 300 saccharides, and more preferably not larger than 200 saccharides.

Examples of the salt of the sugar chain compound not smaller than heptadecasaccharides include salts exemplified as salts of the aforementioned oligosaccharides.

An activity to promote HGF production of the agent of the present invention can be measured by comparing the HGF concentration in the culture medium of HGF-producing cells when the sugar chain compounds such as oligosaccharides or a salt thereof are added with that when they are not added, wherein the concentration of HGF can be measured by ELISA. As an HGF-producing cell, MRC-5 and MRC-9 cells, human fetal lung fibroblasts, can be used.

Anti-blood coagulation activity of the sugar chain compound such as oligosaccharide or a salt thereof used in the agent of the present invention for promoting HGF production can be assessed by measuring an activated partial thromboplastin time (APTT) or anti-factor Xa activity according to the known methods that have been used for measuring anti-blood coagulation activity of low-molecular-weight heparin.

LPL releasing activity of the sugar chain compound such as oligosaccharide used in the agent of the present invention for promoting HGF production or a salt thereof can be assessed by administering the sugar chain compound or a salt thereof to a mouse or a rat, taking the plasma after a certain time, and measuring the amount of LPL in the plasma. The amount of LPL in the plasma can be determined by measuring LPL activity, or measuring the mass of LPL protein by ELISA.

Since the agent of the present invention promotes production of HGF, it is effective in preventing or treating various diseases through pharmacological actions of HGF. The agent of the present invention for promoting HGF production has various uses such as liver disease treating agent, kidney disease treating agent, wound healing agent, skin ulcer treating agent, hair root cell proliferating agent, anti-cancer agent, lung injury treating agent, and side effect preventing agent in cancer therapy. More specifically, the agent of the present invention is useful in preventing or treating diseases such as liver diseases (e.g. hepatitis, cirrhosis, liver failure, liver regeneration after surgical operation, etc.), kidney diseases (e.g. glomerular nephritis, renal insufficiency, renal anemia, diabetic nephropathy, renal damage after drug administration, etc.), skin diseases (e.g. leukoplakia, burn, decubitus, skin ulcer, alopecia, etc.), blood diseases(e.g. thrombocytopenia, bone marrow transplantation, etc.), eye diseases (e.g. corneal ulcer, etc.), lung diseases (e.g. pneumonia, emphysema, pulmonary, tuberculosis, chronic obstructive lung disease, pneumoconiosis, lung fibrosis, etc.), stomach or duodenum diseases (e.g. gastritis, stomach ulcer, duodenumulcer, etc.), cancer diseases and related diseases (e.g. various cancers, side effect in cancer therapy such as hepatic toxicity, renal toxicity, nausea, vomiting, thrombocytopenia, hair loss, etc.), bone diseases (e.g. osteoporosis, bone dysplasia, osteoarthritis, etc.), and diseases of the central nervous system (e.g. abnormality in nerve differentiation, etc.).

The agent of the present invention for promoting HGF production may take various preparation forms (e.g. injections, solid preparations, capsules, etc.), and it is preferable that the oligosaccharide or a salt thereof is formulated into injections together with a conventional carrier. The injection can be prepared by a conventional method, and concentration of the oligosaccharide or a salt thereof in the injection can be appropriately adjusted from a range of about 0.0001 to 5 w/v %. The injection can be prepared, for example, by dissolving the oligosaccharide or a salt thereof in an appropriate solvent (e.g. sterilized water, buffer, physiological saline, etc.), filtering the solution with a filter under sterile conditions, and filling the solution into an aseptic container. A preferable conventional carrier includes stabilizing agents such as albumin, globulin, gelatin, mannitol, glucose, dextran, ethylene glycol, and the like. Further, the oligosaccharide or a salt thereof may be dissolved in a solution containing pharmaceutically acceptable additives necessary for the formulation, such as an isotonic (e.g. sodium chloride, potassium chloride, glycerin, mannitol, sorbitol, boric acid, borax, glucose, propylene glycol, etc.), a buffer (e.g. phosphate buffer, acetate buffer, borate buffer, carbonate buffer, citrate buffer, Tris buffer, glutamate buffer, ε-aminocaproate buffer, etc.), a preservative (methyl paraoxybenzoate, ethyl paraoxybenzoate, propyl paraoxybenzoate, butyl paraoxybenzoate, chlorobutanol, benzyl alcohol, bnezalkonium chloride, sodium dehydroacetate, sodium edetate, boric acid, borax, etc.), a thickener (hydroxyethylcellulose, hydroxypropylcellulose, polyvinyl alcohol, polyethylene glycol, etc.), a stabilizing agent (sodium hydrogen sulfite, sodium thiosulfate, sodium edetate, sodium citrate, ascorbic acid, dibutylhydroxytoluene, etc.), or a pH adjusting agent (hydrochloric acid, sodium hydroxide, phosphoric acid, acetic acid, etc.), and the resultant solution is filtered with a filter under sterile conditions, then filled into an aseptic container. In addition, an appropriate solubilizer such as alcohols (e.g. ethanol, etc.), polyalcohols (e.g. propylene glycol, polyethylene glycol, etc.), and non-ionic surfactants (e.g. polysorbate 80, polyoxyethylene hardened castor oil 50, etc.) may be used. The formulated injectable solution is usually filled into an appropriate ample or vial. It is desirable to store liquid preparations such as injections under freezing conditions or lyophilized conditions. The lyophilized preparation can be redissolved in injectable distilled water before use.

In addition, the agent of the present invention for promoting HGF production may be formulated into external preparations in the form of ointments, gels or liquids. The external preparation can be prepared, for example, by kneading an oligosaccharide or a salt thereof into an ointment base (e.g. absorptive ointment, hydrophilic ointment, macrogol, purified lanolin, etc.). Also, hardening agents (e.g. cetanol, stearyl alcohol, etc.) may be used in the formulation of such external preparations, and in the case of gel preparations, thickeners (e.g. carmellose, hydroxypropylcellulose, povidone, etc.) may be used. Effective ingredient content in the external preparations may be appropriately adjusted to about 0.0001 to 5 w/w % depending on the kinds of diseases, and application site of the external agent, etc.

The agent of the present invention for promoting HGF production may be administered via a suitable route depending on their dosage form. For example, injections may be administered via intravenous, intraarterial, subcutaneous or intramuscular route and the like. The injection dose may be appropriately adjusted depending on the condition, age, and body weight of patients, etc. The injection dose is usually 0.0001 mg to 500 mg, preferably 0.01 mg to 100 mg, which is appropriately administered once a day or several times a day in a divided manner, although it may be appropriately adjusted depending on the condition, age, and body weight of patients, etc.

The agent of the present invention for promoting HGF production may be a mixed preparation of the sugar chain compounds such as oligosaccharides or a salt thereof and HGF. Since it is known that plasma half life of HGF is extended (Kato, Y. et al., Hepatology, 1994, vol.20, p.417 to 424) when HGF in the form of a complex with heparin or heparan sulfate is administered into a living body, formulation of the sugar chain compounds such as oligosaccharides or a salt thereof and HGF into a mixed preparation makes it possible to extend the plasma half life of HGF contained in the mixed preparation. In this case, a blending ratio of an oligosaccharide or a salt thereof to HGF is usually 0.00002 to 50,000 (w/w) (oligosaccharide/HGF), preferably 0.01 to 500 (w/w). Thereupon, since sugar chain compounds such as oligosaccharides or a salt thereof in a preparation promotes production of endogenous HGF in a living body at the same time, it is more effective in treating or preventing diseases through the action of HGF. For the same reason, when the agent of the present invention for promoting HGF production is administered into a living body, it is also possible to administer HGF as another preparation at the same time.

The following Examples further illustrate the present invention in more detail, but the present invention is not limited by these Examples at all.

Each abbreviation used in Examples means as follows:
HGF: hepatocyte growth factor
FCS: fetal calf serum
DMEM medium: Dulbecco's modified Eagle medium
PBS: phosphate buffered saline
ELISA: enzyme-linked immunosorbent assay
MALDI-TOF/MS: Matrix-assisted laser desorption ionization-time of flight mass spectrometry
LPL: lipoprotein lipase
APTT: activated partial thromboplastin time
UA: uronic acid residue
GlcN: glucosamine residue
Ac: acetyl
Arg: arginine
Gly: glycine
Sac: saccharide
2S: 2-O-sulfate
6S: 6-O-sulfate
NS: N-sulfate

### Example 1

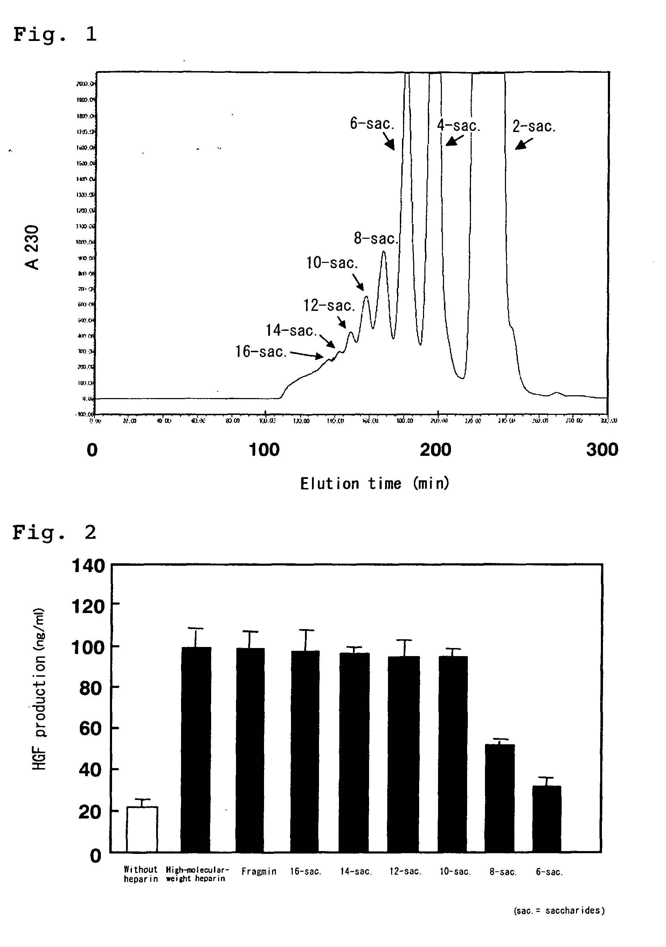

100 mg of high-molecular-weight heparin Na (Scientific Protein Laboratories Inc.) was dissolved in 2 mL of 50 mM sodium acetate buffer (containing 3 mM calcium acetate) (pH 7.0), and 0.5 unit of heparinase (SEIKAGAKU CORPORATION) was added to the mixture to react them at 37°C for 10 hours. The reaction mixture was heated at 100°C for 2 minutes to stop the reaction. 0.3 mL of the supernatant of the reaction mixture was subjected to a Superdex 30pg column (φ1.6 cm × 60 cm) (Amersham Bioscience) to perform gel filtration chromatography. As a mobile phase, 200 mM aqueous ammonium bicarbonate solution was used, and the solution was passed at a flow rate of 0.4 ml/min. Absorbance of the eluate at 230 nm was monitored, and the eluate was fractionated. Heparin fragments were separated and recovered on the basis of molecular size. The heparin fragments were obtained as oligosaccharide fractions of disaccharides, tetrasaccharides, hexasaccharides, octasaccharides, decasaccharides, dodecasaccharides, tetradecasaccharides and hexadecasaccharides (Fig. 1).

### Example 2

Activities to promote HGF production of the heparin fragments (oligosaccharides) obtained in Example 1 were measured by using MRC-9 cells, human fetal lung fibroblasts. The cells were cultured with heparin fragments (oligosaccharides) and amounts of HGF produced by the cell were analyzed. First, MRC-9 cells were seeded onto a 48 well plate at a density of 5 × 10⁴ cells/cm², and cultured in a DMEM medium containing 10% FCS for one day. After removing the medium, the cells were washed twice with 0.5 mL of PBS, and the medium was exchanged with a DMEM medium containing 1% FCS. Thereafter, the culture was continued. At this time, the heparin fragments obtained in Example 1 were added to the medium at a final concentration of 1 µg/ml. After 24 hours, the culture supernatant was recovered, and an amount of HGF secreted into the culture medium was measured by the ELISA method. When a commercially available low-molecular-weight heparin (Fragmin, Kissei Pharmaceutical Co., Ltd.) was added, the amount of HGF produced by the cells was equivalent to that in the case when high-molecular-weight heparin was added, and HGF production level was about 5-fold higher than that in the case without heparin. Also, when heparin fragments not larger than hexadecasaccharide prepared in Example 1 were added, HGF production was increased in heparin fragments not smaller than hexasaccharide compared with the case when heparin was not added (Fig. 2).

### Example 3

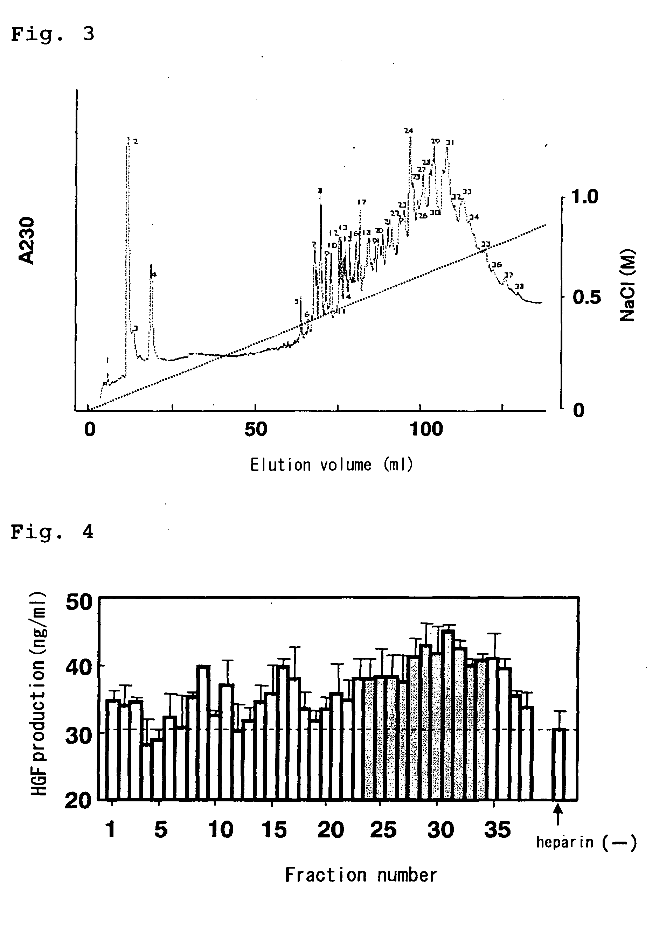

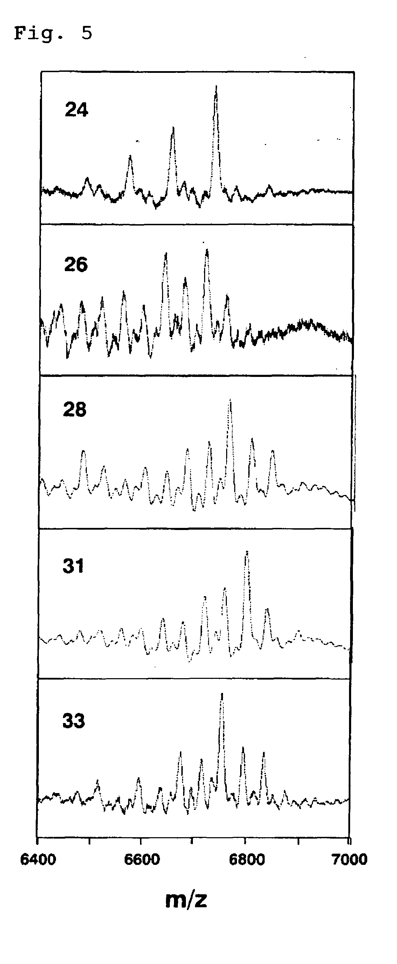

The decasaccharide fraction of heparin fragments (hereinafter referred to as heparin decasaccharide fragments) obtained in Example 1 was freeze-dried and re-dissolved in water. The resulting solution was subjected to a MiniQ4.5/50 column (Amersham Bioscience) to perform anion-exchange chromatography for further separation. The heparin decasaccharide fragments were eluted by linear gradient elution, in which aqueous NaCl was used as an eluent (flow rate: 0.5 ml/min) and the concentration of NaCl was linearly increased from OM to 1M. Absorbance of the eluate at 230 nm was monitored and fractionated (Fig. 3). The heparin decasaccharide fragments were separated as many peaks supposedly due to variation of the number and the positions of sulfate groups, and the individual peaks were recovered. The recovered fractions were numbered 1 to 38, and the activity to promote HGF production of each peak was measured in a similar manner to Example 2 (Fig. 4). The result showed that most of the fractions had the activity to promote HGF production. From these results, it is unlikely that some specific structures in heparin decasaccharide fragments play an essential role in exhibiting the activity to promote HGF production, and therefore it may be considered that the presence of sulfate groups at appropriate positions in the basal structure of heparin decasaccharide fragments that is comprised of repetition of uronic acid and glucosamine is sufficient. However, the activity to promote HGF production is thought to be increased along with the increase in the number of sulfate groups, since the activity to promote HGF production tend to be higher in the later fractions (fraction Nos. 24 to 36).

The numbers of sulfate groups in heparin decasaccharide fragments contained in fraction Nos . 24, 26, 28, 31 and 33 were estimated by MALDI-TOF/MS analysis using a mass spectrometer (UltraFlex; Bruker Daltonics). Each fraction was desalted by dialysis using a dialysis membrane (cut-off molecular weight: 1000), freeze-dried and re-dissolved in water to prepare a sample. In order to neutralize the negative charges of the heparin fragments due to the sulfate groups, a basic peptide (Arg-Gly)₁₉Arg ([M + H]⁺ =4226.8 (theoretical value)) was mixed in a sample, and MALDI-TOF/MS analysis was performed using caffeic acid as a matrix (Fig. 5). Since the heparin fragments are detected as a complex with (Arg-Gly)₁₉Arg in this analysis, the molecular weights of the heparin fragments can be calculated by subtracting the molecular weight of (Arg-Gly)₁₉Arg from the detected value of m/z (mass/charge). Plural m/z signals were detected in all fractions, which showed that plural components coexisted in the fractions. The m/z signal pattern was shifted to a higher-molecular side along with the increase in the fraction number (from 24 to 33), and thus it could be confirmed that oligosaccharides in the fraction of a larger number had more sulfate groups. The compositions of the fractions Nos. 24 to 33 estimated from the molecular weights are shown in Table 1.

**Table 1**

| Fraction No. | Complex m/z (A) | Pept i de m/z Observed Value (B) | Oligosaccharide Molecular weight (A-B) | ΔUA-GlcNS 415.3 | ΔUA-GlCNA 377.31 | H 1.01 | SO₃H 81. 07 | Total number of SO₃H | Theoretical molecular weight |
|---|---|---|---|---|---|---|---|---|---|
| 24 | 6481.76 | 4227.60 | 2254.16 | 1 | 4 | 6 | 4 | 5 | 2254.91 |
| | 6562.05 | | 2334.45 | 1 | 4 | 5 | 5 | 6 | 2334.97 |
| | 6641. 98 | | 2414. 38 | 1 | 4 | 4 | 6 | 7 | 2415.03 |
| | 6721. 87 | | 2494. 27 | 1 | 4 | 3 | 7 | 8 | 2495. 09 |
| | | | | | | | | | |
| 26 | 6438.53 | 4226.80 | 2211.73 | 2 | 3 | 7 | 3 | 6 | 2212.87 |
| | 6560. 81 | | 2334. 01 | 1 | 4 | 5 | 5 | 7 | 2334.97 |
| | 6641.64 | | 2414.84 | 1 | 4 | 4 | 6 | 8 | 2415.03 |
| | 6679. 42 | | 2452.62 | 2 | 3 | 4 | 6 | 9 | 2453. 05 |
| | 6721.41 | | 2494. 61 | 1 | 4 | 3 | 7 | 9 | 2495. 09 |
| | 6759.56 | | 2532.76 | 2 | 3 | 3 | 7 | 10 | 2533.11 |
| | | | | | | | | | |
| 28 | 6682.56 | 4227.64 | 2454.92 | 2 | 3 | 4 | 6 | 8 | 2453.05 |
| | 6724. 38 | | 2496. 74 | 1 | 4 | 3 | 7 | 8 | 2495.09 |
| | 6761.76 | | 2534.12 | 2 | 3 | 3 | 7 | 9 | 2533.11 |
| | 6804.03 | | 2576. 39 | 1 | 4 | 2 | 8 | 9 | 2575.15 |
| | 6843.51 | | 2615.87 | 2 | 3 | 2 | 8 | 10 | 2613.17 |
| | | | | | | | | | |
| 30 | 6639.35 | 4226.83 | 2412.52 | 3 | 2 | 5 | 5 | 8 | 2411.01 |
| | 6718.81 | | 2491.98 | 3 | 2 | 4 | 6 | 9 | 2491.07 |
| | 6757.28 | | 2530.45 | 4 | 1 | 4 | 6 | 10 | 2529.09 |
| | 6799.13 | | 2572.30 | 3 | 2 | 3 | 7 | 10 | 2571.13 |
| | 6837. 67 | | 2610. 84 | 4 | 1 | 3 | 7 | 11 | 2609.15 |
| | | | | | | | | | |
| 33 | 6677.32 | 4226.85 | 2450.47 | 4 | 1 | 5 | 5 | 9 | 2449.03 |
| | 6717. 37 | | 2490. 52 | 3 | 2 | 4 | 6 | 9 | 2491.07 |
| | 6756.57 | | 2529.72 | 4 | 1 | 4 | 6 | 10 | 2529.09 |
| | 6797.29 | | 2570.44 | 3 | 2 | 3 | 7 | 10 | 2571.13 |
| | 6836.36 | | 2609.51 | 4 | 1 | 3 | 7 | 11 | 2609.15 |

### Example 4

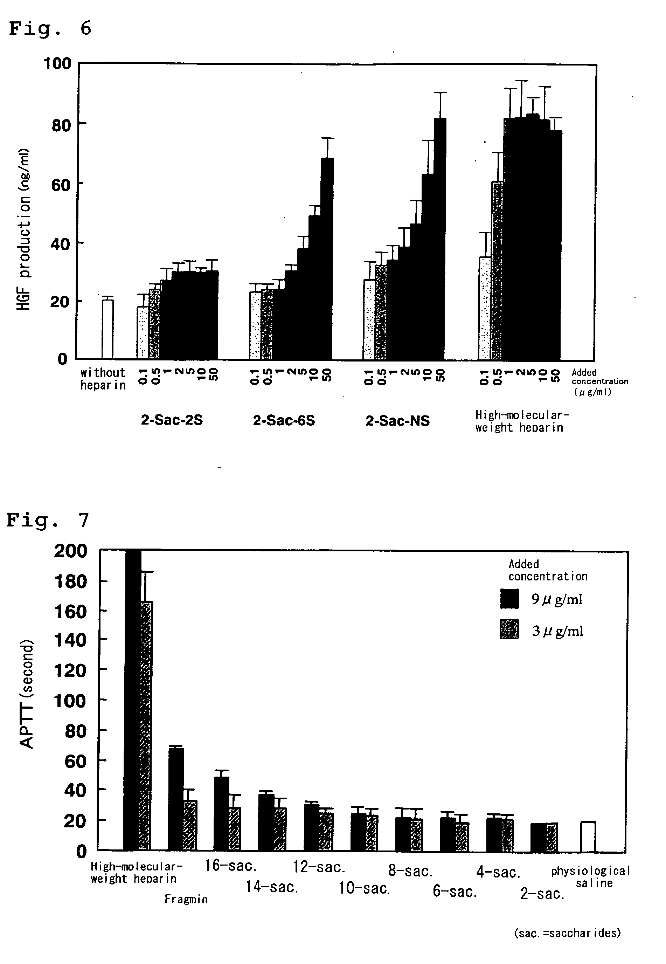

The activity to promote HGF production of the following disaccharide compounds-sulfated at different positions were measured: 2-Sac-2S(Heparin disaccharide III-H; manufactured by Sigma), 2-Sac-6S(ΔDiHS-6S; manufactured by SEIKAGAKU CORPORATION) and 2-Sac-NS(ΔDiHS-NS; manufactured by SEIKAGAKU CORPORATION)(table 2)(Fig. 6). Their activity to promote HGF production was measured using MRC-9 cells as in Example 2. At this time, concentrations of the disaccharide compounds were changed from 0 to 50 µg/ml.

It was proved that all the disaccharide compounds had an activity to promote HGF production though the activity varied depending on the position of a sulfate group and they needed to be added at a higher concentration compared to an unfractionated heparin to exhibit the effect to promote HGF production. 2-Sac-6S and 2-Sac-NS were proved to have a higher activity to promote HGF production than 2-Sac-2S, which indicated that sulfation of the hydroxy group at position 6 and/or the amino group at position 2 of the glucosamine residue was important for exhibiting an activity to promote HGF production.

**Table 2**

| Symbol | Simplified structure | 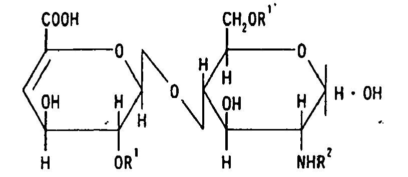 | | |
|---|---|---|---|---|
| | | R¹ | R ² | R^{1'} |
| 2-Sac-2S | ΔUA-2S-GlcN | SO₃H | H | H |
| 2-Sac-6S | ΔUA-GlcNAc-6S | H | COCH₃ | SO₃H |
| 2-Sac-NS | ΔUA-GlcNS | H | SO₃H | H |

### Example 5

Regarding the heparin fragments (oligosaccharides) obtained in Example 1, anti-blood coagulation activity and LPL releasing activity were measured. Anti-blood coagulation activity was assessed by comparing an activated partial thromboplastin time (APTT). APTT was measured using fresh plasma taken from Wister rat (male) and Data φ·APTT kit (Sysmex) which utilizes activation by ellagic acid. The heparin fragments were added to rat plasma at a concentration of 9 µg/mL or 3 µg/mL, and APTT values (second) were compared.

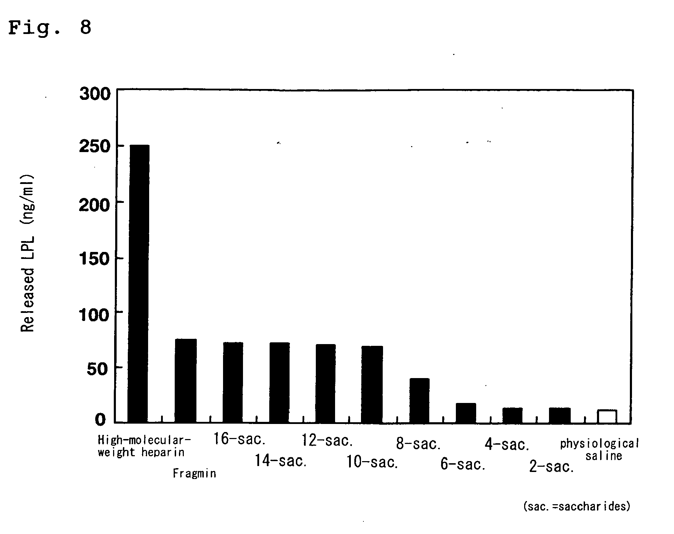

Results of APTT measurement are shown in Fig. 7. When a physiological saline was added in place of heparin, the plasma was rapidly coagulated and APTT was about 20 seconds. When an undigested high-molecular weight heparin was added to the plasma at a concentration of 9 µg/mL, plasma coagulation was inhibited and APTT was extended to 200 seconds or longer. Also when a high-molecular weight heparin was added at a concentration of 3 µg/mL, APTT was 160 seconds or longer, and plasma coagulation was inhibited to a large extent. In the case of commercially available low-molecular weight heparin (Fragmin, Kissei Pharmaceutical Co., Ltd.), extension of APTT was suppressed compared with high-molecular weight heparin, but extension of APTT was still significant level. When the hexadecasaccharide heparin fragment of Example 1 was added at a concentration of 9 µg/mL, APTT was about 46 seconds. Thus APTT was extended compared with that when heparin was not added, but the degree of APTT extension was significantly shorter than Fragmin, indicating that anti-coagulation activity was reduced to a large extent. Further, APTT was shortened with the reduction in molecular size. APTTs of oligosaccharides not larger than tetrasaccharide at either a concentration of 9 µg/mL or 3 µg/mL were equivalent to that when physiological saline was added.

For measuring LPL releasing activity, heparin fragments were administered to Wister rat (3-week old male) via the tail vein at 0.3 µg per 1 g of body weight and, after 10 minutes, blood was taken from axillary fossa venous plexus, and a LPL amount in the resulting plasma was measured by ELISA (Daiichi Pure Chemicals Co., Ltd). Results of measurement of released LPL amount are shown in Fig. 8. In high-molecular weight heparin, about 250 ng/mL LPL was released, but in low-molecular weight heparin (Fragmin, Kissei Pharmaceutical Co., Ltd.), released LPL amount was reduced to about 30% of the amount in the case of high-molecular weight heparin. In the heparin fragments prepared in Example 1, released LPL amount was also reduced with the decrease in molecular size of heparin fragments. In particular, in oligosaccharides not larger than octasaccharide, reduction in released LPL amount was remarkable.

### Example 6

To further examine anti-blood coagulation activity of the heparin decasaccharide fragments obtained in Example 1, anti-factor Xa activity and anti-factor IIa activity were measured.

For the measurement of anti-factor Xa activity, 2.5 µL of antithrombin III (2.5 U) solution was added to 22 , 5 µL of a sample solution, and the mixture was incubated at 37 °C for 3 minutes, and 12.5 µL of anti-blood coagulation factor Xa solution (7.1 nkat S-2222/mL) was added thereto. To the mixed solution, 2.5 µL of a solution of S-2222 (0.75mg/mL)(Daiichi Pure Chemicals Co. , Ltd), a substrate of factor Xa, was added, the mixture was reacted at 37°C for 3 minutes, and the reaction was stopped by addition of 37.5 µL of 50 v/v% aqueous acetic acid. The color intensity was measured by analyzing absorbance at 405 nm. The color development is due to p-nitroaniline liberated from S-2222 in the reaction with factor Xa. The color intensity of a sample containing a substance having an anti-factor Xa activity is decreased compared with that of a sample containing no substance having an anti-factor Xa activity, which means that this decrease (ΔA405) in the color intensity reflects the anti-factor Xa activity in the samples. A calibration curve for anti-factor Xa activity versus ΔA405 was prepared using a standard low-molecular-weight heparin (anti-factor Xa activity: 175U/mg, anti-factor IIa activity 69U/mg; National Institute of Health Science), and the anti-factor Xa activities of tested samples were determined on the basis of the calibration curve. The anti-factor IIa activity was measured similarly using S-1127 (0.625 mg/ml; Daiichi Pure Chemicals Co., Ltd), anti-blood coagulation factor IIa (0.4 U/ml) and 0.01M citric acid, instead of S-2222, factor Xa and 50%(v/v) aqueous acetic acid, respectively.

The results of measurement of anti-factor Xa activity and anti-factor IIa activity are shown in Table 3. The anti-factor Xa activity and the anti-factor IIa activity of Fragmin, a commercially available low-molecular heparin, were decreased to 75% and 32%, respectively, of those of an unfractionated heparin. On the other hand, the anti-factor Xa activity and the anti-factor IIa activity of heparin decasaccharide fragments were significantly decreased to 12% and 4%, respectively, of those of an unfractionated heparin.

**Table 3**

| | Anti-factor Xa activity (U/mg) | Anti-factor IIa activity (U/mg) |
|---|---|---|
| Unfractionated heparin | 203.9 + 24.2 | 184.1 + 24.1 |
| Fragmin | 152.0 + 7.7 | 58.2 + 0.6 |
| Heparin decasaccharide fragments | 24.9 + 0.9 | 8.2 + 2.6 |

### Example 7

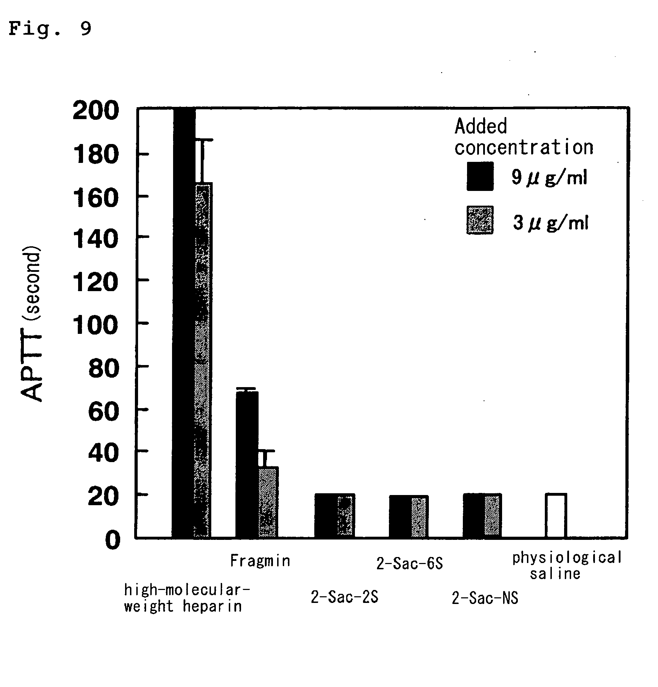

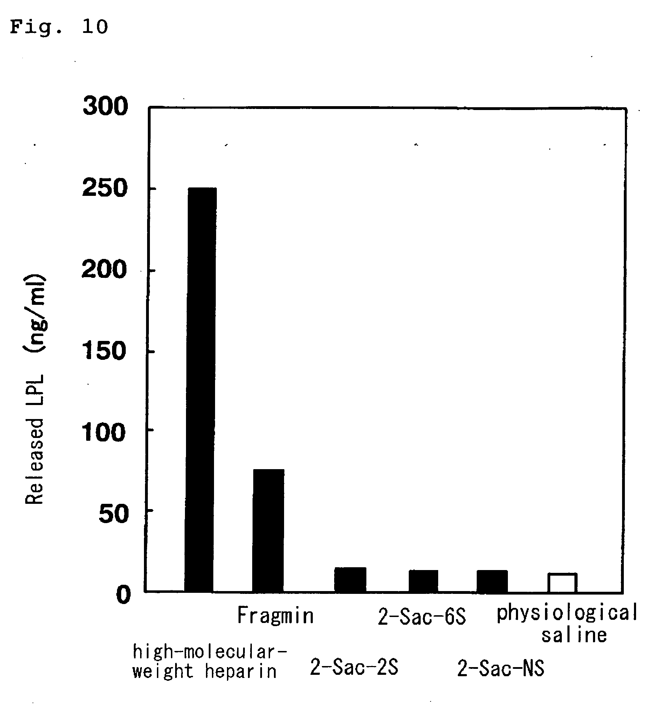

Regarding the disaccharide compounds sulfated at different positions, such as 2-Sac-2S (Sigma), 2-Sac-6S (SEIKAGAKU CORPORATION), and 2-Sac-NS (SEIKAGAKU CORPORATION) described in Example 4, anti-blood coagulation activity and LPL releasing activity were measured by the method similar to Example 5 (Fig. 9 and Fig. 10). In all the disaccharide compounds, anti-blood coagulation activity and LPL releasing activity were not detected.

### Example 8

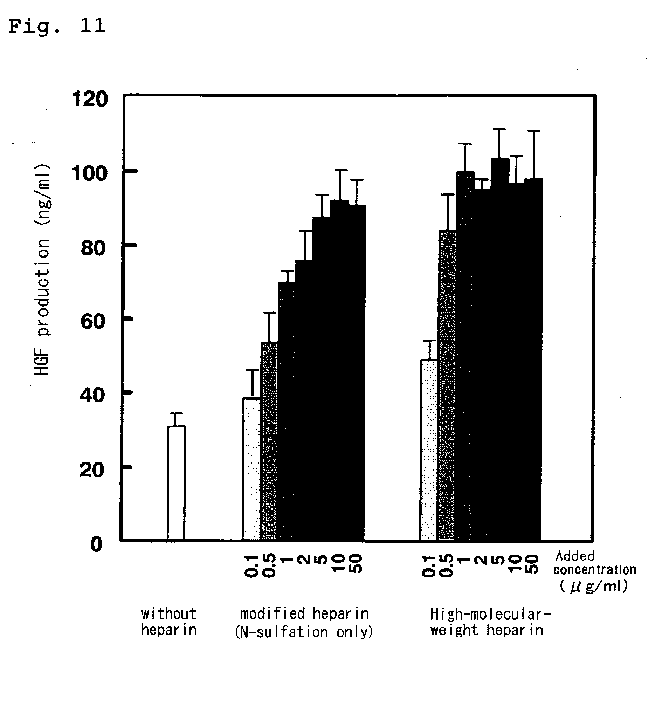

Regarding a modified heparin (CDSNS-Heparin manufactured by SEIKAGAKU CORPORATION) in which only the amino group at position 2 of glucosamine was sulfated (N-sulfation only), the activity to promote HGF production was measured using MRC-9 cells by the method similar to Example 2 (Fig. 11). In this measurement, the concentration of the modified heparin, in which only the amino group at position 2 of glucosamine was sulfated, was varied in the range of 0 to 50 µg/mL. Although the HGF production-promoting activity of the modified heparin, in which only the amino group at position 2 of glucosamine was sulfated, was slightly weaker than that of an unfractionated heparin, its maximum activity was almost equal to that of a normal unfractionated heparin.

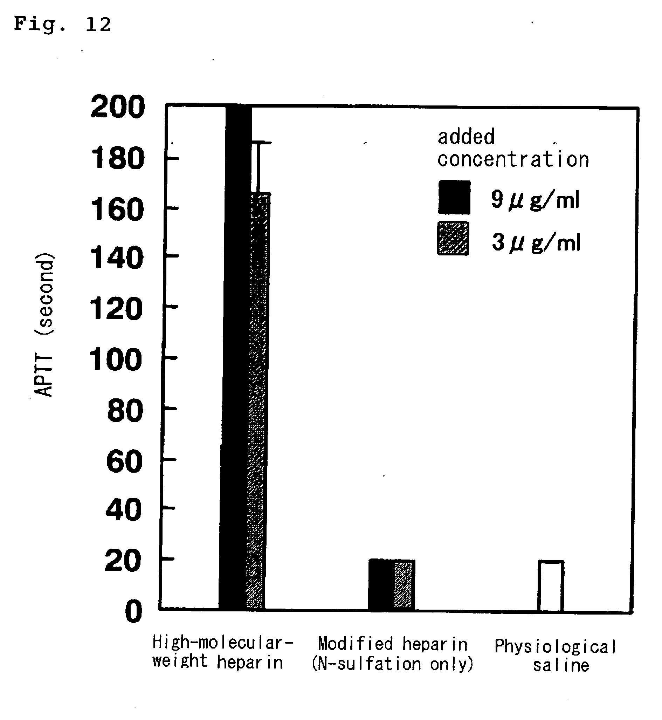

Also, in order to examine an anti-blood coagulation activity of the modified heparin in which only the amino group at position 2 of glucosamine was sulfated, APTT was measured by the method similar to Example 5 (Fig. 12). In the modified heparin in which only the amino group at position 2 of glucosamine was sulfated, APTT was not extended and thus no anti- blood coagulation activity was detected.

### INDUSTRIAL APPLICABILITY

The agent of the present invention for promoting HGF production can promote HGF production with suppressed side effect of bleeding tendency, and is useful to promote healing of damaged tissues or organs of a living body.

## Claims

1. An agent for promoting HGF production comprising, as an effective ingredient, a disaccharide comprised of an uronic acid residue (wherein an uronic acid means an iduronic acid or a glucuronic acid, and has the same meaning hereinafter) and a glucosamine residue that are connected by α1,4-glycosidic linkage or β1,4-glycosidic linkage, or tri- to hexadeca-saccharides having a structure in which uronic acid residue(s) and glucosamine residue(s) are alternately and repeatedly connected by α1,4-glycosidic linkage or β1,4-glycosidic linkage, wherein at least one hydroxy group of the uronic acid residue(s) and/or the glucosamine residue(s) may be sulfated, alkylated, acylated or aminated, and/or amino group at position 2 of at least one of the glucosamine residue(s) may be sulfated, alkylated or acylated, or a salt thereof.

2. The agent for promoting HGF production according to claim 1, wherein the hydroxy group at position 2 of at least one of the uronic acid residue(s) and/or the hydroxy group at positions 3 and/or 6 of at least one of the glucosamine residue(s) may be sulfated.

3. The agent for promoting HGF production according to claim 1 or 2, wherein the hydroxy group at position 6 and/or the amino group at position 2 of at least one of the glucosamine residue (s) is sulfated.

4. The agent for promoting HGF production according to any one of claims 1 to 3, wherein the oligosaccharide is di- to deca-saccharide.

5. The agent for promoting HGF production according to any one of claims 1 to 4, wherein the oligosaccharide is a degradation product prepared from high-molecular-weight heparin or high-molecular-weight heparan sulfate by digestion with heparinase or heparitinase.

6. The agent for promoting HGF production according to any one of claims 1 to 4, wherein the oligosaccharide is a degradation product prepared from high-molecular-weight heparin or high-molecular-weight heparan sulfate by digestion by any one of nitrous acid degradation, hydrogen peroxide degradation or β-elimination.

7. The agent for promoting HGF production according to claim 1, wherein the oligosaccharide is any one of compounds represented by the following (a) to (h);
(a) formula (I): 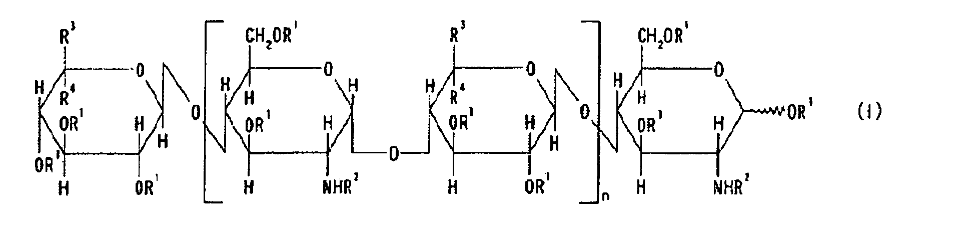 wherein R¹ represents hydrogen, sulfate group, alkyl, acyl, or optionally substituted amino group, R² represents hydrogen, sulfate group, alkyl or acyl group, R³ and R⁴ are different from each other and represent hydrogen or optionally substituted carboxyl group, and n represents 0 to 7,
(b) formula (II): 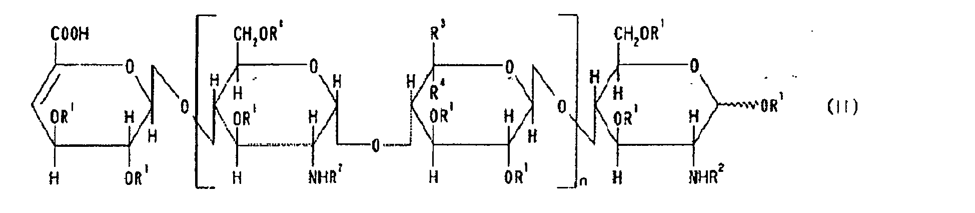 wherein all the symbols are respectively the same as defined above,
(c) formula (III): 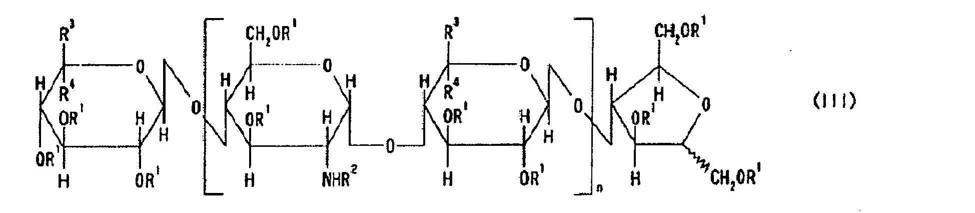 wherein all the symbols are respectively the same as defined above,
(d) formula (IV): 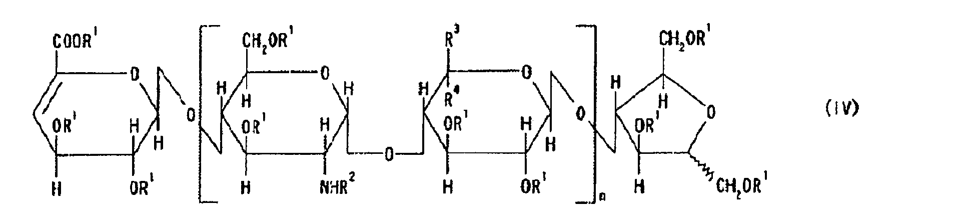 wherein all the symbols are respectively the same as defined above,
(e) formula (V): 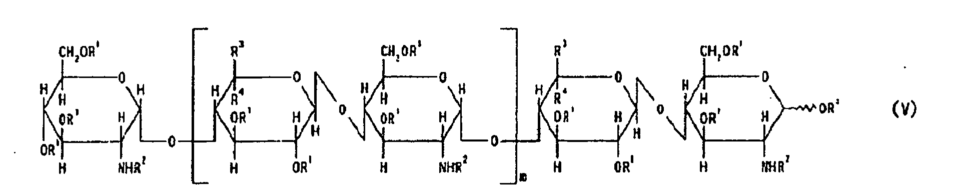 wherein m represents 0 to 6, and R¹, R², R³ and R⁴ are respectively the same as defined above,
(f) formula (VI): 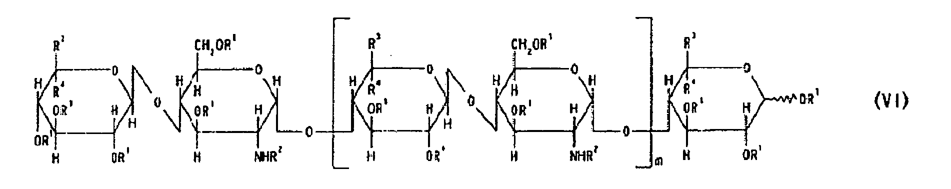 wherein m represents 0 to 6, and R¹, R², R³ and R⁴ are respectively the same as defined above,
(g) formula (VII) 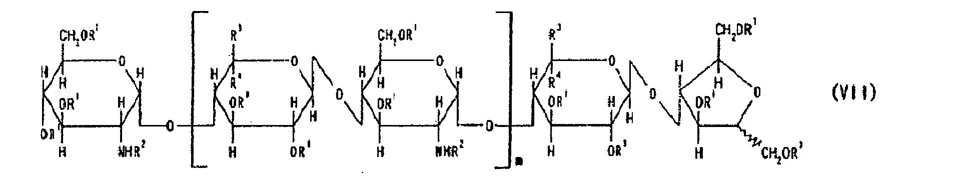 wherein m represents 0 to 6, and R¹, R², R³ and R⁴ are respectively the same as defined above, and
(h) formula (VIII) 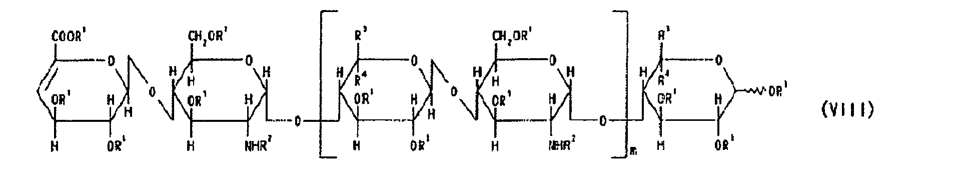
wherein m represents 0 to 6, and R¹, R², R³ and R⁴ are respectively the same as defined above.

8. An agent for promoting HGF production comprising, as an effective ingredient, a sugar chain compound having a structure in which uronic acid residue(s) and glucosamine residue(s) are alternately and repeatedly connected by α1,4-glycosidic linkage or β1,4-glycosidic linkage, wherein the hydroxy group at position 6 of at least one of the glucosamine residue(s) is sulfated, or a salt thereof.

9. An agent for promoting HGF production comprising, as an effective ingredient, a sugar chain compound having a structure in which uronic acid residue(s) and glucosamine residue(s) are alternately and repeatedly connected by α1,4-glycosidic linkage or β1,4-glycosidic linkage, wherein the amino group at position 2 of at least one of the glucosamine residue(s) is sulfated, or a salt thereof.

10. An agent for promoting HGF production comprising, as an effective ingredient, a disaccharide compound comprised of an uronic acid residue and a glucosamine residue wherein the hydroxy group at position 6 of the glucosamine residue and/or the amino group at position 2 of the glucosamine residue are/is sulfated are connected by α1,4-glycosidic linkage or β1,4-glycosidic linkage, or a salt thereof.

11. The agent for promoting HGF production according to any one of claims 1 to 10, wherein the sugar chain compound or a salt thereof has no or reduced anti-blood coagulation activity and/or lipoprotein lipase releasing activity.

12. A method of promoting HGF production **characterized by** administering to a mammal an effective amount of a disaccharide composed of an uronic acid residue (wherein an uronic acid means an iduronic acid or a glucuronic acid, and has the same meaning hereinafter) and a glucosamine residue that are connected by α1,4-glycosidic linkage or β1,4-glycosidic linkage, or tri- to hexadeca-saccharides having a structure in which uronic acid residue(s) and glucosamine residue(s) are alternately and repeatedly connected by α1,4-glycosidic linkage or β1,4-glycosidic linkage, wherein at least one hydroxy group of the uronic acid residue(s) and/or the glucosamine residue(s) may be sulfated, alkylated, acylated or aminated, and/or the amino group at position 2 of at least one of the glucosamine residue(s) may be sulfated, alkylated or acylated, or a salt.

13. The method of promoting HGF production according to claim 12, wherein the hydroxy group at position 2 of at least one of the uronic acid residue(s) and/or the hydroxy group at positions 3 and/or 6 of at least one of the glucosamine residue(s) may be sulfated.

14. The method of promoting HGF production according to claim 12, wherein the hydroxy group at position 6 and/or the amino group at position 2 of at least one of the glucosamine residue(s) is sulfated.

15. The method of promoting HGF production according to claim 12, wherein the oligosaccharide is di- to deca-saccharide.

16. The method of promoting HGF production according to claim 12, wherein the oligosaccharide is a degradation product prepared from high-molecular-weight heparin or high-molecular-weight heparan sulfate by digestion with heparinase or heparitinase.

17. The method of promoting HGF production according to claim 12, wherein the oligosaccharide is a degradation product prepared from high-molecular-weight heparin or high-molecular-weight heparan sulfate by any one of nitrous acid degradation, hydrogen peroxide degradation or β-elimination.

18. The method of promoting HGF production according to claim 12, wherein the oligosaccharide is any one of compounds represented by the following (a) to (h);
(a) formula (I): 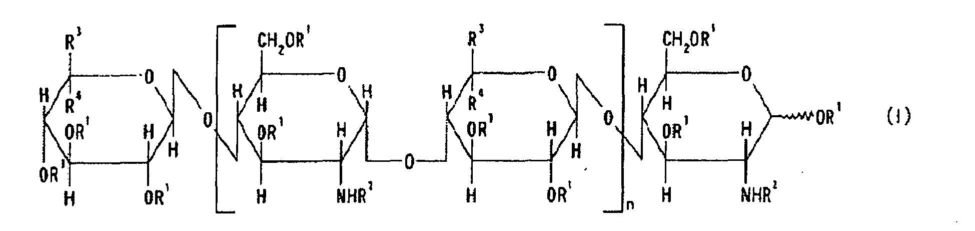 wherein R¹ represents hydrogen, sulfate group, alkyl, acyl, or optionally substituted amino group, R² represents hydrogen, sulfate group, alkyl or acyl group, R³ and R⁴ are different from each other and represent hydrogen or optionally substituted carboxyl group, and n represents 0 to 7,
(b) formula (II): 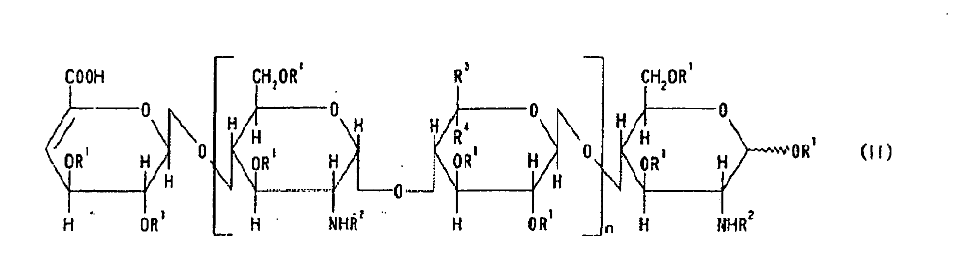 wherein all the symbols are respectively the same as defined above,
(c) formula (III): 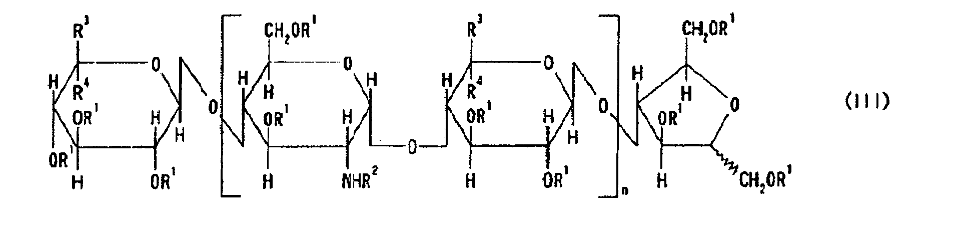 wherein all the symbols are respectively the same as defined above,
(d) formula (IV): 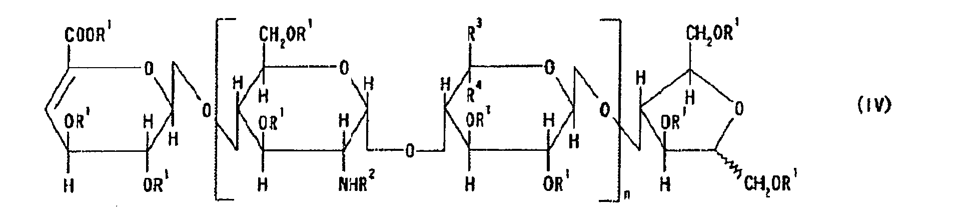 wherein all the symbols are respectively the same as defined above,
(e) formula (V): 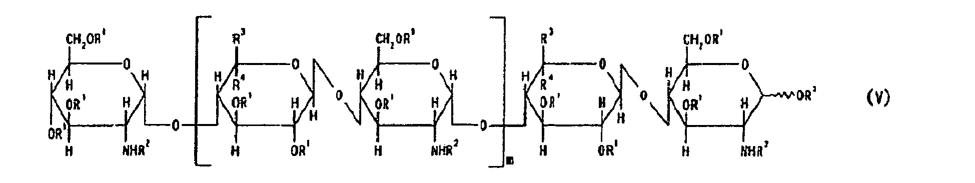 wherein m represents 0 to 6, and R¹, R², R³ and R⁴ are respectively the same as defined above,
(f) formula (VI): 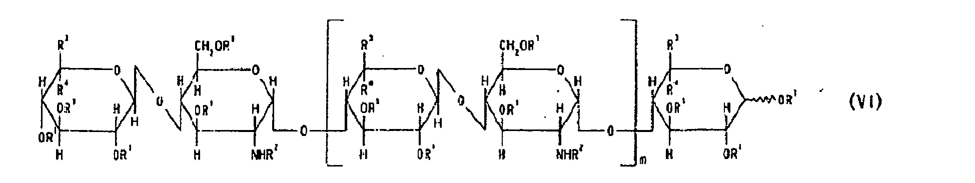 wherein m represents 0 to 6, and R¹, R², R³ and R⁴ are respectively the same as defined above,
(g) formula (VII) 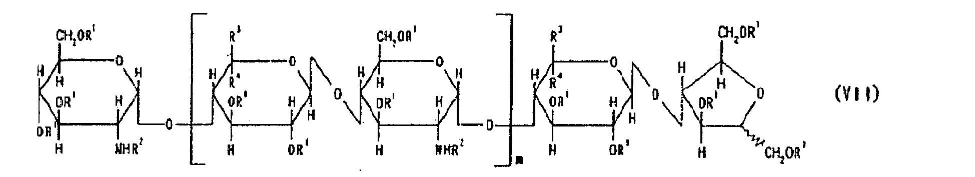 wherein m represents 0 to 6, and R¹, R², R³ and R⁴ are respectively the same as defined above, and
(h) formula (VIII) 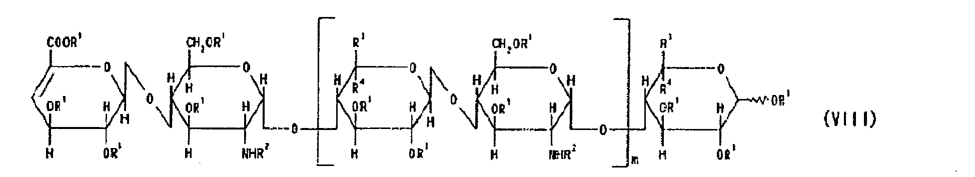
wherein m represents 0 to 6, and R¹, R², R³ and R⁴ are respectively the same as defined above.

19. A method of promoting HGF production **characterized by** administering to a mammal an effective amount of a sugar chain compound having a structure in which uronic acid residue (s) and glucosamine residue(s) are alternately and repeatedly connected by α1,4-glycosidic linkage or β1,4-glycosidic linkage, wherein the hydroxy group at position 6 of at least one of the glucosamine residue(s) is sulfated, or a salt thereof.

20. A method of promoting HGF production **characterized by** administering to a mammal an effective amount of a sugar chain compound having a structure in which uronic acid residue(s) and glucosamine residue(s) are alternately and repeatedly connected by α1,4-glycosidic linkage or β1,4-glycosidic linkage, wherein the amino group at position 2 of at least one of the glucosamine residue(s) is sulfated, or a salt thereof.

21. A method of promoting HGF production **characterized by** administering to a mammal an effective amount of a disaccharide compound comprised of an uronic acid residue and a glucosamine residue in which the hydroxy group at position 6 and/or the amino group at position 2 of the glucosamine residue are/is sulfated are connected by α1,4-glycosidic linkage or β1,4-glycosidic linkage, or a salt thereof.

22. The method of promoting HGF production according to any one of claims 12 to 21, wherein the sugar chain compound or a salt thereof has no or reduced anti-blood coagulation activity and/or lipoprotein lipase releasing activity.

23. Use of a disaccharide composed of an uronic acid residue(wherein an uronic acid means an iduronic acid or a glucuronic acid, and has the same meaning hereinafter) and a glucosamine residue that are connected by α1,4-glycosidic linkage or β1,4-glycosidic linkage, or tri- to hexadeca-saccharides having a structure in which uronic acid residue(s) and glucosamine residue(s) are alternately and repeatedly connected by α1,4-glycosidic linkage or β1,4-glycosidic linkage, wherein at least one hydroxy group of the uronic acid residue(s) and/or the glucosamine residue(s) may be sulfated, alkylated, acylated or aminated, and/or the amino group at position 2 of at least one of the glucosamine residue(s) may be sulfated, alkylated or acylated, or a salt thereof, for the production of a medicament for promoting HGF production.

24. The use according to claim 23, wherein the hydroxy group at position 2 of at least one of the uronic acid residue (s) and/or the hydroxy group at positions 3 and/or 6 of at least one of the glucosamine residue(s) may be sulfated.

25. The use according to claim 23, wherein the hydroxy group at position 6 and/or the amino group at position 2 of at least one of the glucosamine residue(s) is sulfated.

26. The use according to claim 23, wherein the oligosaccharide is di- to deca-saccharide.

27. The use according to claim 23, wherein the oligosaccharide is a degradation product prepared from high-molecular-weight heparin or high-molecular-weight heparan sulfate by digestion with heparinase or heparitinase.

28. The use according to claim 23, wherein the oligosaccharide is a degradation product prepared from high-molecular-weight heparin or high-molecular-weight heparan sulfate by any one of nitrous acid degradation, hydrogen peroxide degradation or β-elimination.

29. The use according to claim 23 wherein the oligosaccharide is any one of compounds represented by the following (a) to (h);
(a) formula (I): 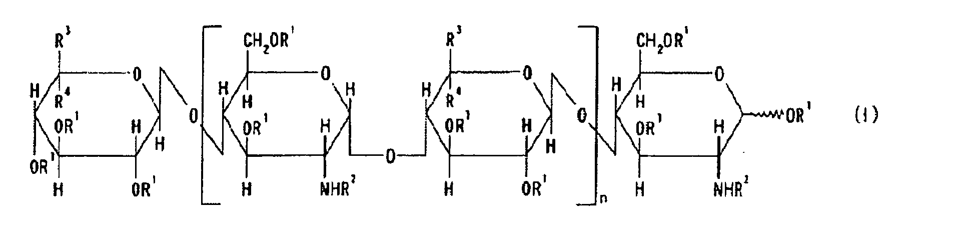 wherein R¹ represents hydrogen, sulfate group, alkyl, acyl, or optionally substituted amino group, R² represents hydrogen, sulfate group, alkyl or acyl group, R³ and R⁴ are different from each other and represent hydrogen or optionally substituted carboxyl group, and n represents 0 to 7,
(b) formula (II): 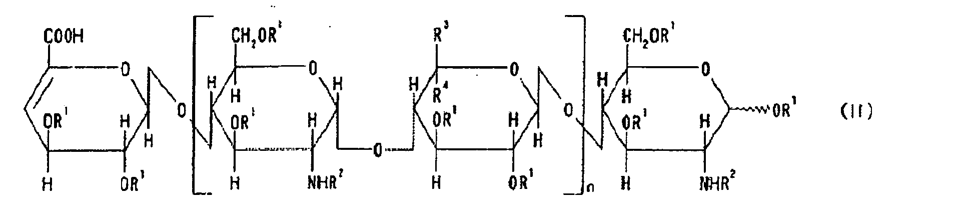 wherein all the symbols are respectively the same as defined above,
(c) formula (III): 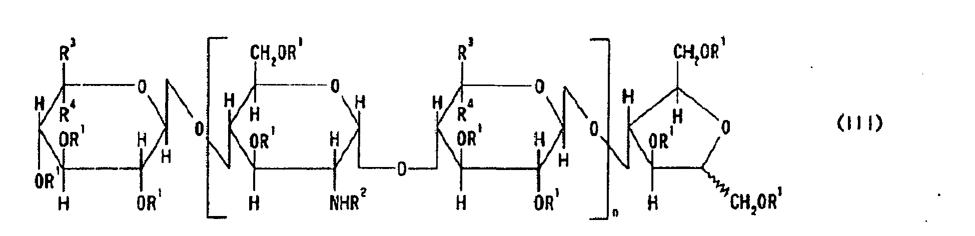 wherein all the symbols are respectively the same as defined above,
(d) formula (IV): 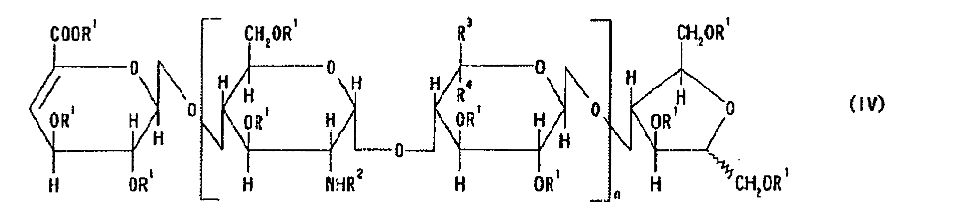 wherein all the symbols are respectively the same as defined above,
(e) formula (V): 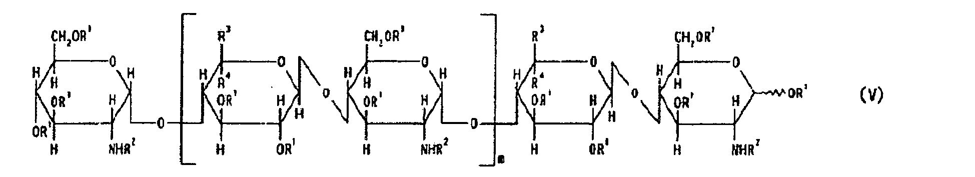 wherein m represents 0 to 6, and R¹, R², R³ and R⁴ are respectively the same as defined above,
(f) formula (VI): 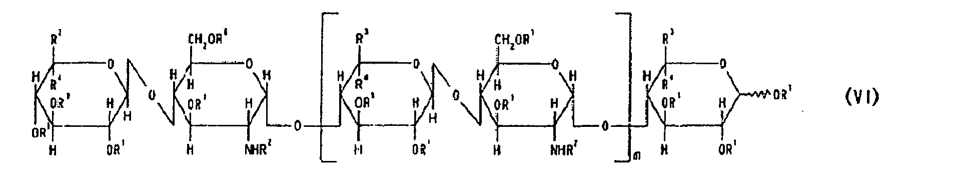 wherein m represents 0 to 6, and R¹, R², R³ and R⁴ are respectively the same as defined above,
(g) formula (VII) 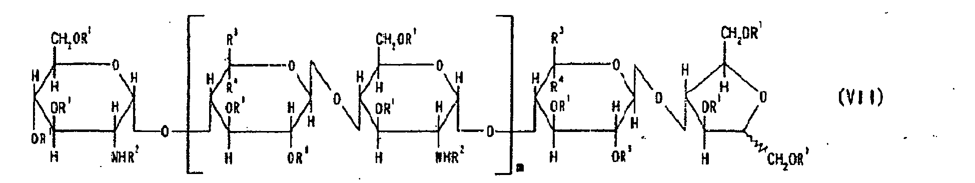 wherein m represents 0 to 6, and R¹, R², R³ and R⁴ are respectively the same as defined above, and
(h) formula (VIII) 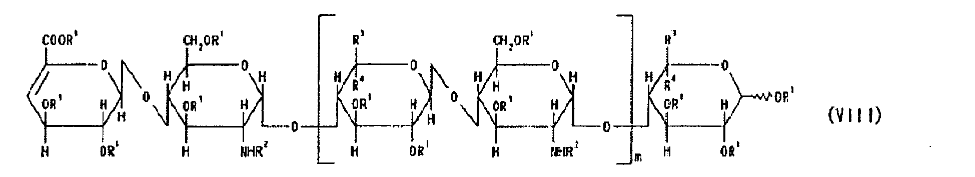
wherein m represents 0 to 6, and R¹, R², R³ and R⁴ are respectively the same as defined above.

30. Use of a sugar chain compound having a structure in which uronic acid residue(s) and glucosamine residue(s) are alternately and repeatedly connected by α1,4-glycosidic linkage or β1,4-glycosidic linkage, wherein the hydroxy group at position 6 of at least one of the glucosamine residue(s) is sulfated, or a salt thereof, for the production of a medicament for promoting HGF production.

31. Use of a sugar chain compound having a structure in which uronic acid residue(s) and glucosamine residue(s) are alternately and repeatedly connected by α1,4-glycosidic linkage or β1,4-glycosidic linkage, wherein at least one amino group at position 2 of the glucosamine residue(s) is sulfated, or a salt thereof, for the production of a medicament for promoting HGF production.

32. Use of a disaccharide compound comprised of an uronic acid residue and a glucosamine residue wherein the hydroxy group at position 6 and/or the amino group at position 2 of the glucosamine residue are/is sulfated are connected by α1,4-glycosidic linkage or β1,4-glycosidic linkage, or a salt thereof, for the production of a medicament for promoting HGF production.

33. The use according to any one of claims 23 to 32, wherein the sugar chain compound or a salt thereof has no or reduced anti-blood coagulation activity and/or lipoprotein lipase releasing activity.
